# EUROPEAN PATENT APPLICATION

(11) **EP 3 692 989 A1**
(43) Date of publication of application: **12.08.2020**
(21) Application number: 20164705.4
(22) Date of filing: 04.06.2009
(51) Int. Cl.: A61K 31/4035, A61P 25/00

(54) **USE OF ISOINDOLES FOR THE TREATMENT OF NEUROBEHAVIORAL DISORDERS**

(30) Priority: 20.06.2008 US 74500
(62) Divisional of application: 16200855.1
(71) Applicant: Afecta Pharmaceuticals, Inc., Irvine, CA 92612 (US)
(72) Inventor: KOVACS, Bruce, Long Beach, California 90803 (US); PINEGAR, Laura, Long Beach, California 90803 (US)
(74) Representative: Petty, Catrin Helen

(57) **Abstract**

The present invention generally relates to the use of drugs for the treatment of neurobehavioral disorders or symptoms of a neurobehavioral disorder associated with dysfunction of the trimonoamine modulating system (TMMS). More specifically, the invention describes methods for the treatment of a neurobehavioral disorder and/or treatment or prevention of symptoms of a neurobehavioral disorder by administering suitable Isoindole derivatives alone or in combination with other agents so as to provide relatively equal inhibitory effect on serotonin, dopamine and norepinephrine transporters.

## Description

### CROSS-REFERENCE TO RELATED APPLICATIONS

The present application claims priority to prior U.S. provisional application Serial Number 61/074,500, filed on June 20, 2008, the entire contents of which are hereby incorporated by reference.

### TECHNICAL FIELD

The present invention generally relates to the use of drugs for the treatment of neurobehavioral disorders or symptoms of a neurobehavioral disorder associated with dysfunction of the trimonoamine modulating system (TMMS). More specifically, the invention describes methods for the treatment of a neurobehavioral disorder and/or treatment or prevention of symptoms of a neurobehavioral disorder by administering suitable Isoindole derivatives alone or in combination with other agents so as to provide relatively equal inhibitory effect on serotonin, dopamine and norepinephrine transporters.

### BACKGROUND OF THE INVENTION

Taken collectively, neurobehavioral disorders affect a significant percentage of the population. These disorders range from those conditions that first manifest in early childhood to those that occur exclusively in adults. Although clinically they manifest with disparate symptomatology, the underlying etiology reflects dysfunction in one or more basic neuronal circuits in the central nervous system (CNS). Neurobehavioral disorders can be inherited or acquired and the actual manifestation of the disorder is influenced by genetic diatheses, the individual's environment and other circumstances, such as physical changes.

Within the CNS, five basic functional neuronal circuits are described; the brainstem, the hypothalamic, the motor striatal, the limbic striatal and the neocortical striatal. All neurobehavioral disorders can be regarded as occurring when one or more of these five brain circuits become dysfunctional. From the perspective of the neuroanatomical substrates all neurobehavioral disorders involve such structures as: prefrontal cortex, cingulate cortex, entorhinal cortex, hippocampus, nucleus accumbens (ventral striatum), ventral pallidum, amygdala, and anterior hypothalamus (see Swanson and Petrovich, 1998, Kalivas et al., 1993 and Heimer, 2003). Connections between these structures form the complex neuronal circuits noted above. Furthermore, projections between structures are organized in a strict topographical manner (See e.g. van Groen et al., 2002 and Heidbreder and Groenewegen, 2003). The resulting functional macrostructure is primarily responsible for the generation and expression of motivational, cognitive and affective states, as well as motor activity. In addition, because of shared neurobiological substrates many primarily neurobehavioral disorders involve abnormal or involuntary movements such as those present in obsessive-compulsive disorder, frontotemporal dementia, and attention deficit-hyperactivity disorder. Similarly many disorders which primarily manifest through abnormal motor function such as Tourette syndrome and Huntington's disease also have neurobehavioral, psychiatric and/or cognitive manifestations as well.

The trimonoamine modulating system (TMMS), comprising the dopaminergic (DA), serotonergic (5HT) and noradrenergic (NE) nuclei, modulate the activities of the five basic circuitries note above (Othmer & Othmer et al, 1998). This occurs as a result of the modulatory effects of the TMMS on fast excitatory and inhibitory synaptic transmission in these circuits.

Fast synaptic transmission in the five basic nueronal circuits is mediated by ligand gated ion channels. Glutamate (GLU), is the primary fast excitatory neurotransmitter of the CNS, and gamma-amniobutyric acid (GABA) is the primary fast inhibitory neurottransmitter of the CNS. An important ligand gated ion channel active by glutamate is the NMDA receptor family, while the primary target of GABA is ligand gated chloride channel In contrast, all three monoamines members of the TMMS (domamine, serotonin and norepinephrine) use G-protein coupled receptors and use second messenger cytoplasmic pathways. Therefore, they can interact synergetically or antagonistically, depending on the secondary messengers involved. For example, in the case of noradrenaline and serotonin, depletion of one neurotransmitter has little effect on patients treated with an antidepressant acting primarily on the other neurotransmitter. Also in contrast to glutamate and GABA, the monoamines of the TMMS have a striking organization in the brain: the cell bodies producing the monoamines are restricted to a small number of nuclei, but their axons project widely throughout the nervous system.

Therefore, it is possible that the TMMS may act to modulate fast synaptic transmission mediated by glutamate and gamma-amniobutyric acid through the extensive projections of their axons throughout the CNS. In support of this concept is the fact that virtually all antidepressants reduce ligand binding to glutamatergic NMDA receptors in the frontal cortex. Thus, modulation of NMDA receptor activation probably underlies the antidepressant action of both serotonergic and noradrenergic reuptake inhibitors. In chronically depressed patients, a reduction of glial cells in the subgenual prefrontal cortex (a small region connected to the hypothalamus and situated beneath the genua, or knee, of the corpus callossum), has been suspected to result in inadequate glutamate transport leading to receptor activation. Similarly, GABAergic neurons, which constitute about 95% of the neurons in the striatal complex area, form an inhibitory synaptic network that is modulated by dopamine and perhaps other monoamines.

Thus, drive-related activities mediated by the hypothalamus, emotional/motivational activities mediated by the limbic circuitry and value-based behaviors, cognition in the neocortex circuitry as well as motor and reward based behaviors may all be modulated by the TMMS, through fine control of the; brainstem, hypothalamic, motor-striatal, limbic-striatal and the neocortical striatal circuits. Therefore, if there is dysfunction, imbalance or damage to the TMMS, the modulatory effects could be altered and conversely, if the fast synaptic transmission in the five neuronal circuits is abnormal it could be corrected by manipulation of the TMMS through simultaneous and differential alteration of the synaptic levels of the three monoamines; DA, 5HT and NE. In addition, if there is a dysfunction in the TMMS the deficient and/or abnormal modulation of the effected substrate CNS circuitries would result in the clinical manifestation of various neurobehavioral disorders.

As discovered by the inventors and disclosed herein, many otherwise unrelated neurobehavioral disorders in humans are associated with dysfunction of the TMMS. These include; attention deficit disorder (ADD) attention deficit disorder with hyperactivity (ADHD), and other Pervasive Developmental Disorders (such as Autism, Asperger's disorder and Rett's syndrome), obsessive compulsive disorders (OCD), post traumatic stress disorder (PTSD), other Anxiety Disorders (such as phobias and panic disorder), bipolar disorder, dysthymia and other mood disorders, Tourette syndrome and other movement disorders, somatoform disorders like body dysmorphic disorder and substance abuse disorders. Heretofore, there has been no underlying common pathophysiologic mechanism which could explain such apparently disparate neurobehavioral disorders and no single pharmacologic agent which would have effects on all such disorders through a common neurophysiologic substrate. This finding has application in the novel treatment of such disorders.

Currently, many of the drugs used in the treatment of these disorders selectively modulate or alter serotonergic or dopaminergic neurotransmission (Jones and Blackburn, 2002). None modulate all three monoamine of the TMMS , simultaneously and to a relatively equal degree. However, in order to achieve desired effects in the range of disorders associated TMMS dysfunction it would be highly desirable to use a single molecule (or combination of molecules) that simultaneously and differentially modulate all three monoamines of the TMMS to a relatively equal degree. Much like controlling the level of sound in the; base, mid, and treble frequency ranges within a given range of amplitude using a sound "mixer" in a high fidelity audio system can result in better overall sound quality for different types of music, the simultaneous and differential modulation of the monoamines in the TMMS within a given range of efficiency can result in fine control of fast and slow excitatory neurotransmission and result more precise modulation of multiple neuronal circuits, resulting in therapeutic affects on neurobehaviors associated with different functional regions of the brain. As disclosed herein, the end result is a fine tuning of neuronal circuitry through simultaneous modulation of DA, 5HT and NE activity in the TMMS that are involved with neurobehavioral disorders, which heretofore is unprecedented.

As disclosed herein, the inventors have surprisingly discovered that the various, and heretofore unrelated categories of neurobehavioral behavioral disorders disclosed herein all have as a part of their basic neuropathology the dysfunction of the TMMS as demonstrated by the association of polymorphic genetic variation in the genes encoding the trimonoamine transporters. In addition, the inventors have also discovered that the range of compounds according to formula (I) have significant clinical utility in treatment of a variety of neurobehavioral disorders by the modulation of the levels of all three monoamines of the TMMS simultaneously and differentially within a specified range of efficiency. The inventors have discovered that the dysfunction in the TMMS underlying many seemingly unrelated neurobehavioral disorders can be corrected to achieve a clinically useful therapeutic effect by the simultaneous and relatively equal inhibitory effect on the dopamine, serotonin and norepinephrine transporters. More specifically, the inventors have found that the members of the class of compounds contemplated within the scope of the present invention which have a highly selective inhibitory effect on the dopamine transporter (DAT) and which, at the same time have a highly selective inhibitory effect on the serotonin transporter (SERT) as well as having a highly selective inhibitory effect on the on the norepinephrine transporter (NET) such that the inhibitory effect as measured by the Ki at each of these transporter is relatively equal (i.e. the Ki ratio: DAT:SERT, DAT:NET, and SERT: NET is less than 50:1) show a unique effect in treating underlying dysregulation various neuronal circuits and resulting neurobehavioral disorders via action on the TMMS

The consequence of this unique capacity results in an improved ability to treat individuals with a variety of neurobehavioral disorders including two or more disorders occurring at the same time in an individual, as well as a single disorder occurring in a much larger population of individuals which has only been possible previously using a combination of compounds.

Isoindole derivatives having useful pharmaceutical activity in the areas of depression, and as anorexic agents in the treatment of obesity are disclosed in U.S. Pat. Nos; 3,444,181; 4,075,351; 4,513,006; 4,591,601; 4,792,569, and 5,760,007. One of these compounds 5-(p-chlorophenyl)-2,5,-dihydro-3H-imidazo(2,1,a)isoindol-5-ol (known as Mazindol) has been used clinically as an appetite suppressant. The use of Mazindol has also been disclosed in US Pat. No. 3,966,955 for the treatment of gastric ulcer, in US Pat. No. 5,447,948 for the treatment of the negative symptoms of schizophrenia and in U.S. Pat No. 5,217,987 to reduce cocaine craving. Epstein discloses in United States Patent Application 20020161002 the use of mazindol to enhance long-term memory in several disorders including ADHD. However, it is not disclosed nor is it obvious that mazindol alone would be useful in treating the specific behavioral symptoms of; inattention, hypereactivity, and impulsivity without the need for other medications.

Mazindol has also been reported in the treatment of Duchenne muscular dystrophy (Griggs, et al. 1990), narcolepsy (Alvarez, et al. 1991), and urinary incontinence.

### SUMMARY OF THE INVENTION

It is an object of the present invention to provide for the use of Isoindole derivatives for the treatment of abnormal behavioral symptoms in a wide range of disorders by the relatively equal and simultaneous differential modulation of the three monoamines of the TMMS.

The present invention comprises methods for the treatment or prevention of neurobehavioral disorders using the compounds of formula (I), or pharmaceutical compositions containing one or more of the compounds of formula (I), or pharmaceutical compositions containing one or more pro-drugs of the compounds of formula (I), or one or more of the compounds of formula (I) as well as a general method of treatment of neurobehavioral disorders by simultaneous modulation DA, 5HT and NE comprising the chemical effectors of the TMMS, by the simultaneous and relatively equal inhibition of their transporters in the CNS, which can also be used in addition to a safe and effective amount of one or more other agents to treat related symptoms and conditions.

### DETAILED DISCRIPTION OF PREFERED EMBODIMENTS

### DEFINITIONS

"Attention Deficit Hyperactivity Disorder" as used herein, refers to an individual demonstrating any one or all of the behaviors and characteristics associated with attention deficit disorder with hyperactivity (ADHD). More specifically, as used herein ADHD refers a persistent pattern of difficulties resulting in one or more of the following behaviors: inattention, hyperactivity and impulsivity. Inattention as used herein refers to difficulty attending to or focusing on a specific task, staying organized, keeping track of time, completing tasks, and/or making frequent errors in cognitive tasks. Hyperactivity as used herein refers to difficulty inhibiting motor behavior such that there may be constant motion, excessive fiddling, leg swinging, and/or squirming and similar motor action. Impulsivity as used herein refers to prone to act on impulse causing difficulty in controlling behaviors. Thus there are inappropriate actions, blurring out, impatience, and lack of self control. As used herein the present invention will us the term "Attention Deficit Hyperactivity Disorder" to refer to an individual demonstrating any or all of the behaviors and characteristics associated with ADHD as defined herein

"Autism Spectrum Disorder" as used herein, refers to an individual demonstrating any one or all of the symptoms and characteristics associated with autism. Such individual may fit particular diagnostic criteria, such as; Autistic Disorder, Asperger's Disorder, Atypical Autism or Pervasive Developmental Disorder, NOS (not otherwise specified), Rett's Disorder or Childhood Disintegrative Disorder, and the broader autism phenotype disorder or such individual may not fit a discrete diagnostic category at all. Due to the many presentations of the condition called autism, the present invention will use the term "autism" to refer to an individual demonstrating any one or all of the behavioral symptoms and characteristics associated with "Autism" and/or all of the above disorders.

"Dementia Disorder" as used herein, dementia refers to a state characterized by loss of function in multiple cognitive domains such as impairment of; reasoning, planning, personality, social cognition and communication or executive function. More specifically, dementia as used herein refers to an individual demonstrating abnormal behaviors such as; aphasia, apraxia, and/or agnosia or loss of the normal abilities of; reasoning, planning, social cognition and communication or executive function. The dementia disorder man occur in an individual with a specific known neuropathophysiologic cause such as; neurological AIDS, syphilis, Alzheimer's disease, Creutzfeld-Jakob's syndrome, bovine spongiform encephalopathy (BSE) or other prion related infections, diseases involving mitochondrial dysfunction, diseases involving .beta.-amyloid and/or tauopathy such as Down's syndrome, hepatic encephalopathy, Huntington's disease, multiple sclerosis (MS), olivoponto-cerebellar atrophy, post-operative dementia (POCD), Parkinson's dementia, mild cognitive impairment, dementia pugilisitca, vascular and frontal lobe dementia, Lewy body dementia, hypoglycaemia, hypoxia (e.g. perinatal), ischaemia (e.g. resulting from cardiac arrest, stroke, bypass operations or transplants), glioma and other tumors, causing dementia, Korsakoff syndrome, vascular dementia, (including that resulting from Borna virus infection), treatment-induced dementia (e.g. resulting from chemotherapy or radiotherapy), and delirium and combinations thereof, or such individual may not fit a discrete diagnostic category at all. As used herein the present invention will us the term "Dementia Disorder" to refer to an individual demonstrating any or all of the symptoms and characteristics associated with dementia as defined herein including when such dementia occurs as a result of and/or all of the above specific causes.

"Compounds" refers to compounds encompassed by generic formulae disclosed herein, any subgenus of those generic formulae, and any specific compounds within those generic or subgeneric formulae. The compounds can be specific specie, a subgenus or larger genus identified either by their chemical structure and/or chemical name. Further, compounds also include substitutions or modifications of any of such species, subgenuses or genuses, which are set forth herein. When the chemical structure and chemical name conflict, the chemical structure is determinative of the identity of the compound. The compounds can contain one or more chiral centers and/or double bonds and therefore, can exist as stereoisomers, such as double-bond isomers (i.e., geometric isomers), enantiomers or diastereomers. Accordingly, the chemical structures within the scope of the specification encompass all possible enantiomers and stereoisomers of the illustrated compounds including the stereoisomerically pure form (e.g., geometrically pure, enantiomerically pure or diastereomerically pure) and enantiomeric and stereoisomeric mixtures. Further, when partial structures of the compounds are illustrated, asterisks indicate the point of attachment of the partial structure to the rest of the molecule. Enantiomeric and stereoisomeric mixtures can be resolved into their component enantiomers or stereoisomers using separation techniques or chiral synthesis techniques well known to the skilled artisan.

"Impulse Control Disorder" as used herein refers to an individual demonstrating any or all of the behaviors and characteristics of poor impulse control. Such an individual may fit a particular diagnostic criteria, such as; intermittent explosive disorder, kleptomania, pyromania, pathologic gambling, tricotillomania and combinations thereof, or such individual may not fit a discrete diagnostic category at all. As used herein impulse control disorder may be caused by other underlying medical conditions. As used herein the present invention will use the term "Impulse control disorder(s)" to refer to an individual demonstrating the behaviors and characteristics of poor impulse control and/or all of the above disorders

"Individual" as used herein refers to a; person, human adult or child, mammal, or non-human primate.

"IC50" as used herein refers to the molar concentration of a compound or compounds according to formula 1 which inhibits 50% of the monoamine uptake in vitro.

"Ki" as used herein refers to the kinetic inhibition constant in molar concentration units which denotes the affinity of a compound or compounds according to formula 1 for the dopamine, serotonin or norepinephrine transporter as measured by a binding assay or as calculated from the IC50 value using the Cheng-Prusoff equation.

"Movement Disorder" as used herein refers to an individual demonstrating any or all of the behaviors and characteristics of abnormal uncontrollable movements. The movement disorder in an individual may have a specific known pathophysiologic cause associated with the abnormal uncontrollable movements such as; Benign Essential Tremor, Huntingtons disease, Tourette's syndrome, Restless Leg syndrome, Tardive dyskinesia, Myoclonus, Tic's or Sydeham's chorea, or such individual may not fit a discrete diagnostic category at all. As used herein the present invention will use the term "Movement Disorder" to refers to an individual demonstrating the symptoms and characteristics of abnormal involuntary movement due to wide variety of disease states and physiological conditions and/or all of the above disorders

"Monoamines" as used herein refers to serotonin, dopamine and norepinephrine present in the trimonoamine modulating system.

"Neurobehavioral disorder" as used herein refers to a; person, human adult or child, mammal, or non-human primate manifesting the symptoms or characteristics of; "Attention Deficit Hyperactivity Disorder", "Autism Spectrum Disorder", "Anxiety Disorder", "Cognitive Disorder", "Impulse Control Disorder", "Movement Disorder", "Obsessive-Compulsive Spectrum Disorder", "Pervasive Neurodevelopmental Disorder", "Reward Deficiency Disorder", and "Somatization Disorder" as defined herein.

"Obsessive-Compulsive Spectrum Disorder" as used herein refers to an individual demonstrating any or all of the behaviors and characteristics associated with obsessive compulsive behavior. Such an individual may fit particular diagnostic criteria, such as; Obsessive-Compulsive Disorder, Tourette's syndrome, Sydeham's chorea, torticollis,, body dismorphic disorder, hypochondriasis, eating disorders, impulse control disorders, paraphilias and non-paraphilic sexual addictions, impulse control disorder including intermittent explosive disorder, kleptomania, pathological gambling, pyromania, compulsive shopping, compulsive buying, repetitive self-mutilation, onychophagia, psychogenic excoriation, trichotillomania and combinations thereof, or such individual may not fit a discrete diagnostic category at all. As used herein the present invention will use the term "Obsessive-Compulsive Spectrum Disorder" to refer to an individual demonstrating any or all of the symptoms and characteristics associated with obsessive compulsive behavior as defined herein, and/or to all of the above disorders.

"Patient" as used herein refers to a mammal, e.g., a human, mouse, rat, guinea pig, dog, cat, horse, cow, pig, or non-human primate, such as a monkey, chimpanzee, baboon or rhesus.

"Personality Disorder" as used herein, refers to the class of mental disorders characterized by rigid and on-going patterns of thought and action. More specifically, as used herein, personality disorder refers to an individual demonstrating any one or all of the behaviors and characteristics associated with a personality disorder as defined as Axis II disorders in the Diagnostic and Statistical Manual 4th ed. (DSM-IV-TR). Personality disorders include specific diagnostic categories, such as; borderline personality disorder, schizotypal personality disorder, antisocial personality disorder, narcissistic personality disorder, paranoid personality disorder and mild mental retardation or may be otherwise unclassified. As used herein the the present invention will use the term "Personality Disorder" to refers to an individual demonstrating any one or all of the behaviors and characteristics associated with and/or all of the above diagnostic categories or otherwise unclassified personality disorders.

"Pervasive Neurodevelopmental Disorder" (PND) as used herein, refers to an individual demonstrating any one or all of the behaviors and characteristics associated with pervasive neurodevelopmental dysfunction or delay. PND's are typically characterized by distortions in the development of basic neurological and psychological functions that are involved in;
cognitive, motor, and social skills, such as attentional and perceptual processing, motor movement, executive function, inhibitory controls (e.g., sensory gating), social cognition and communication, and afflictive behaviors. As used herein Pervasive Neurodevelopmental Disorder may be associated with specific chromosomal or gene defects such as Fragile X syndrome, Down syndrome, Angelman syndrome, Beckwith-Wiedemann syndrome or may be associated with craniocerebral malformations such as microcephally, craniodynostosis, lissencephally or be associated with cerebral palsy or may have no identifiable causation. Such an individual may fit particular diagnostic criteria, such as autism, Asperger's disorder, Rett's disorder, attention deficit disorder, or attention deficit-hyperactivity disorder or such individual may not fit a discrete diagnostic category at all. As used herein the present invention will use the term "Pervasive Neurodevelopmental Disorder" to refer to an individual demonstrating any one or all of the behaviors and characteristics associated with pervasive neurodevelopmental dysfunction or delay and/or all of the above disorders

"Pharmaceutically acceptable" as used herein means approved by a regulatory agency of the Federal or a state government or listed in the U.S. Pharmacopeia or other generally recognized pharmacopeia for use in animals, and more particularly in humans.

"Pharmaceutically acceptable salt" refers to a salt of a compound of the invention that is pharmaceutically acceptable and that possesses the desired pharmacological activity of the parent compound. Such salts include but are not limited to: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like.

"Pharmaceutically acceptable vehicle" refers to a diluent, adjuvant, excipient or carrier with which a compound of the invention is administered.

"Preventing" or "prevention" refers to a reduction in risk of acquiring a disease or disorder (i.e., causing at least one of the clinical symptoms of the disease not to develop in a patient that may be exposed to or predisposed to the disease but does not yet experience or display symptoms of the disease).

"Prodrug" refers to a derivative of a drug molecule that requires a transformation within the body to release the active drug. Prodrugs are frequently (though not necessarily) pharmacologically inactive until converted to the parent drug. Typically, prodrugs are designed to overcome pharmaceutical and/or pharmacokinetically based problems associated with the parent drug molecule that would otherwise limit the clinical usefulness of the drug.

"Promoiety" refers to a form of protecting group that when used to mask a functional group within a drug molecule converts the drug into a prodrug. Typically, the promoiety will be attached to the drug via bond(s) that are cleaved by enzymatic or non-enzymatic means in vivo. Ideally, the promoiety is rapidly cleared from the body upon cleavage from the prodrug.

"Protecting group" refers to a grouping of atoms that when attached to a reactive group in a molecule masks, reduces or prevents that reactivity. Examples of protecting groups can be found in Green et al., "Protective Groups in Organic Chemistry" , (Wiley, 2.sup.nd ed. 1991) and Harrison et al., "Compendium of Synthetic Organic Methods", Vols. 1 8 (John Wiley and Sons, 1971 1996). Representative amino protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("SES"), trityl and substituted trityl groups, allyloxycarbonyl, 9-fluorenylmethyloxycarbonyl ("FMOC"), nitro-veratryloxycarbonyl ("NVOC") and the like. Representative hydroxy protecting groups include, but are not limited to, those where the hydroxy group is either acylated or alkylated such as benzyl, and trityl ethers as well as alkyl ethers, tetrahydropyranyl ethers, trialkylsilyl ethers and allyl ethers.

"Relatively equal inhibitory effect" as used herein refers to the situation in which the Ki of a compound or compounds according to formula 1 at the dopamine, serotonin and norepinephrine transporters does not exceed a ratio of 50:1 at any of these transporters relative to the other two transporters.

"Reward Deficiency Disorder" as used herein refers to an individual demonstrating the behaviors and characteristics of reward deficiency. Such an individual may fit particular diagnostic criteria, such as; pathological gambling, sexual addiction, schizoid/avoidant personality and combinations thereof, or such individual may not fit a discrete diagnostic category at all. As used herein the present invention will use the term "Reward Deficiency Disorders" to refer to an individual demonstrating the symptoms and characteristics of reward deficiency and/or all of the above specific disorders.

"Somatization Disorder" as used herein refers to an individual demonstrating any or all of the behaviors and characteristics of somatization. Such an individual may fit particular diagnostic criteria, such as; somatoform disorder, conversion disorder, pain disorder, fibromyalgia, chronic fatigue, multiple chemical sensitivity syndrome, hypochrondriasis, body dysmorphic disorder, dysthymia, chronic irritable bowel syndrome and combinations thereof, or such individual may not fit a discrete diagnostic category at all. As used herein, Somatization disorder may be caused by other underlying medical conditions or chronic infections such as but not limited to; multiple sclerosis, acquired immune deficiency syndrome, and various cancers. As used herein the present invention will use the term "Somatization Disorders" to refer to an individual demonstrating the symptoms and characteristics of somatization and/or all of the above disorders

"Treating" or "treatment" of any disease or disorder as used herein, refers, in one embodiment, to ameliorating the disease or disorder (i.e., arresting or reducing the development of the disease or at least one of the clinical symptoms thereof). In another embodiment "treating" or "treatment" refers to ameliorating at least one physical parameter, which may not be discernible by the patient. In yet another embodiment, "treating" or "treatment" refers to inhibiting the disease or disorder, either physically, (e.g., stabilization of a discernible symptom), physiologically, (e.g., stabilization of a physical parameter), or both. In yet another embodiment, "treating" or "treatment" refers to delaying the onset of the disease or disorder.

"Therapeutically effective amount" as used herein, means the amount of a compound that, when administered to an individual for treating a disease, is sufficient to effect such treatment for the disease or to achieve the desired clinical response. The "therapeutically effective amount" will vary depending on the compound, the disease and its severity and the age, weight, etc., of the patient to be treated.

### DETAILED DESCRIPTION

Reference will now be made in detail to preferred embodiments of the invention. While the invention will be described in conjunction with the preferred embodiments, it will be understood that it is not intended to limit the invention to those preferred embodiments. To the contrary, it is intended to cover alternatives, modifications, and equivalents as may be included within the spirit and scope of the invention as defined by the appended claims.

The Isoindole derivatives of use in the present invention are of the following formula (I): 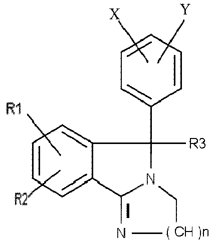 wherein: n is 1 ; R.sub.1 and R.sub.2 are each independently a member selected from the group consisting of hydrogen, halogen, hydroxy, lower alkyl, and lower alkoxy; R.sub.3 is a member selected from the group consisting of hydrogen, halogen, hydroxy, lower alkyl, and lower alkoxy; X and Y each are independently a member selected from the group consisting of hydrogen, halogen, hydroxy, lower alkyl, lower alkoxy, and haloalkyl; or a pharmaceutically acceptable salt, addition compound or pro-drug thereof.

The compounds of formula I: can be prepared by several processes that are per se known to those skilled in the art including processes disclosed in U.S. Pat. Nos. 3,444,181; 3,852,303; 3,930,009; and 3,910,947, which are incorporated in their entirety herein by reference.

Examples of specific compounds of formula (I) are:
3H-Imidazo[2,1-a]isoindol-5-ol, 5-(p-chlorophenyl)-2,5-dihydro- (8CI)
5-(4-Chlorophenyl)-2,5-dihydro-5H-imidazo(2,1-a)isoindol-5-ol
3H-Imidazo[2,1-a]isoindol-5-ol, 5-(3,4-dichlorophenyl)-2,5-dihydro- (8CI,9CI)
5H-Imidazo[2,1-a]isoindole, 5-(3,4-dichlorophenyl)- (8CI,9CI)
5H-Imidazo[2,1-a]isoindole, 5-(4-chlorophenyl)- (9CI)
3H-Imidazo[2,1-a]isoindol-5-ol, 2,5-dihydro-5-[4-(trifluoromethyl)phenyl]- (9CI)
3H-Imidazo[2,1-a]isoindol-5-ol, 5-(3-fluorophenyl)-2,5-dihydro- (9CI)
3H-Imidazo[2,1-a]isoindol-5-ol, 2,5-dihydro-5-phenyl- (8CI,9CI)
3H-Imidazo[2,1-a]isoindol-5-ol, 7,8-dichloro-2,5-dihydro-5-phenyl- (8CI,9CI)
3H-Imidazo[2,1-a]isoindol-5-ol, 5-(3,5-dichlorophenyl)-2,5-dihydro- (8CI,9CI)
5-(4-Chlorophenyl)-2,5-dihydro-5H-imidazo(2,1-a)isoindole
5-(4-chlorophenyl)-8-methyl-5H-imidazo[2,1-a]isoindol-5-ol
5-(3-chloro-4-methoxyphenyl)-5H-imidazo[2,1-a]isoindole
5-(4-chloro-3-methylphenyl)-5H-imidazo[2,1-a]isoindol-5-ol
5-(4-chlorophenyl)-5-methoxy-5H-imidazo[2,1-a]isoindole
5-(3-chloro-5-(chloromethyl)phenyl)-5H-imidazo[2,1-a]isoindole
5-(3-hydroxyphenyl)-5H-imidazo[2,1-a]isoindol-5-ol
and the pharmaceutically acceptable salts thereof.

The compounds of formula I include the various individual steroisomers, diasteteromer, conformational isomers, etc., as well as the racemates and pro-drugs thereof.

In treating a neurobehavioral disorder, a compound of formula (I) may be employed for example at a daily dosage in the range of about 0.01 to 2000 mg administered orally, for an average adult human. It is recognized by those skilled in the art that the exact dosage may be adjusted depending on the severity of symptoms, body weight of the individual and/or other clinical circumstances existing in a given individual. Moreover, it is also recognized that dosage may be adjusted when the compounds according formula (I) are used in combination with other pharmacologically active substances.

To prepare the pharmaceutical compositions of this invention, one or more Isoindole derivatives compounds of formula (I) are intimately admixed with a pharmaceutically acceptable vehicle carrier according to conventional pharmaceutical compounding techniques, which may take a wide variety of forms depending on the form of preparation desired for administration (e.g., oral, transdermal, transmucosal, buccal, nasal, rectal, vaginal, parenteral). In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules and tablets, suitable carriers and additives include starches, sugars, diluents, granulating agents, lubricants, binders, disintegrating agents and the like. Because of their ease in administration, tablets and capsules represent an advantageous oral dosage unit form, in which case solid pharmaceutical carriers are obviously employed. If desired, tablets may be sugar coated or enteric coated by standard techniques.

In addition, various controlled- release delivery methods, well known to those skilled in the art may be employed to improve bioavailablity, reduce side effects, or transdermal delivery may be facilitated by various permeability enhancers or devises. Suppositories may be prepared, in which case cocoa butter could be used as the carrier. For parenterals, the carrier will usually comprise sterile water, though other ingredients, for example, for purposes such as aiding solubility or for preservation, may be included. Inject able suspensions may also be prepared in which case appropriate liquid carriers, suspending agents and the like may be employed. Mazindol no longer available for administration in the United States. However, it is available in other countries (e.g. Mexico) as round tablets containing 1.0 , 2.5 or 5mg of active agent. The tablets contain the following inactive ingredients: lactose hydrous, pregelatinized starch, microcrystalline cellulose, sodium starch glycolate, magnesium stearate, purified water, carnauba wax, hydroxypropyl methylcellulose, titanium dioxide, polyethylene glycol, synthetic iron oxide, and polysorbate 80.

The pharmaceutical compositions herein will contain, per dosage unit, e.g., tablet, capsule, skin patch, buccal lozenge, nasal spray, powder injection, teaspoonful, suppository and the like from about 0.001 to about 9000 mg of the active ingredient.

Included within the scope of this invention are the various individual anomers, diastereomers and enantiomers as well as mixtures thereof. Such compounds are included within the definition of formula (I). For example the selective use of a particular enantiomer (e.g. R or S) of compounds according to formula (I) to achieve a desired therapeutic effect is contemplated within the scope of the present invention since various enantiomers may have differential affinities for the trimonamine transporters of the TMMS. Also contemplated within the scope of the present invention is the selective combination of various individual isomers, such as enantiomers in specific ratios (e.g 3R:1S) to achieve a therapeutic effect. In addition, the compounds of this invention also include any pharmaceutically acceptable salts, for example: alkali metal salts, such as sodium and potassium; ammonium salts; monoalkylammonium salts; dialkylammonium salts; trialkylammonium salts; tetraalkylammonium salts; and tromethamine salts. Hydrates and other solvates of the compound of the formula (I) are included within the scope of this invention.

Pharmaceutically acceptable salts of the compounds of formula (I) can be prepared by reacting the isoindole derivatives of formula (I) with the appropriate base and recovering the salt.

Included within the scope of this invention are various pro-drugs that may be converted by various physiologic processes into the active drug substance or which otherwise improves the bioavailability and/or pharmacological characteristics of the molecules which are the subject of this invention. It is known to those skilled in the art that such pro-dugs may be created by creating derivatives of formula (I) which may be changed by normal physiologic and/or metabolic processes occurring with the individual into the pharmacologically active molecules according to formula (I) or by combining the isoindole derivatives of formula (I) with another molecule or promoiety so as to enhance or control for example; absorption, distribution, metabolism and/or excretion in a individual.

The invention, therefore, also encompasses prodrugs of the present compounds, which on administration undergo chemical conversion by metabolic processes before becoming active pharmacological substances. In general, such prodrugs will be functional derivatives of the present compounds, which are readily convertible in vivo into the required compound of the Formula I. Prodrugs are any covalently bonded compounds, which release the active parent drug according to Formula I in vivo. In cases in which compounds have unsaturated carbon-carbon double bonds, both the cis (Z) and trans (E) isomers are within the scope of this invention. In cases wherein compounds may exist in tautomeric forms, such as ketoenol tautomers, each tautomeric form is contemplated as being included within this invention whether existing in equilibrium or predominantly in one form. Conventional procedures for the selection and preparation of suitable prodrug derivatives are described, for example, in "Design of Prodrugs", ed. H. Bundgaard, Elsevier, 1985.

Prodrug designs are generally discussed in Hardma et al. (eds.), Goodman and Gilman's The Pharmacological Basis of Therapeutics, 9th ed., pages 11-16 (1996).

A further thourough study of prodrug design is presented in Higuchi et al., Prodrugs as Novel Delivery Systems, vol. 14, ASCD Symposium Series, and in Roche (ed.), Bioreversible Carriers in Drug Design, American Pharmaceutical Associateion and Pergamon Press (1987).

For example, the Isoindoles of formula (I) can be linked, coupled or otherwise attached to another molecule which would facilitate the transport of the Isoindole derivatives across cellular or tissue barriers. For example, gastrointestinal absorption can be enhanced by coupling, linking or attaching to another molecule such as a bile acid derivative or analogues to exploit the intestinal bile acid uptake pathway so as to enhance the intestinal absorption. Examples of such conjugations of a specific drug molecule with a carrier molecule, for example a bile acid, are well known to those familiar with the art. For example, Kramer (Biochim. Biophys. Acta. 1227: 137-154, 1994b) describes the conjugation of bile acids with cholesterol lowering drugs (i.e HMG-CoA reductase inhibitors) for example lovastatin to improve gastrointestinal absorption and to facilitate more specific target organ drug delivery.

In addition, the Isoindole derivatives according to formula (I), can be linked, coupled or otherwise attached to molecules which improve penetration of the blood brain barrier. To wit, coupling, linking or attaching the Isoindole derivatives to an essential fatty acid or vitamin to improve penetration into the central nervous system. Such techniques and a large range of molecules and promoieties which can achieve these effects are well known to those skilled in the art of pharmaceutical science. For example a representative compound according to formula 1; (5-(4-Chlorophenyl)-2,3-dihydro-5-hydroxy-5H-imidazo(2,1-a)isoindole) could be conjugated with N-(4-(((2-Amino-1,4-dihydro-4-oxo-6-pteridinyl)methyl)amino)benzo- yl)-L-glutamic acid (folic acid) by formation of an ester linkage to create a new molecule: 2-(4-((2-amino-4-oxo-1,4-dihydropteridin-6-yl)methylamino)benzamido)-5-(5-(4-chlorophenyl)-3,5-dihydro-2H-imidazo[2,1-a]isoindol-5-yloxy)-5-oxopentanoic acid. This molecule would be a prodrug of the representative compound (5-(4-Chlorophenyl)-2,3-dihydro-5-hydroxy-5H-imidazo(2,1-a)isoindole. The attached folate moiety would serve to facilitate the transport of the representative compound across the intestinal barrier and into the blood stream and/or into the brain via the folate transporter mechanism. After transport, naturally occurring tissue esterases would cleave the prodrug molecule at the ester linkage into folic acid and the active drug molecule (5-(4-Chlorophenyl)-2,3-dihydro-5-hydroxy-5H-imidazo(2,1-a)isoindole). Another example of a prodrug of the compounds according to Formula I is (5-(4-Chloropheny-2,3-dihydro-5-hydroxy-5H-imidazo(2,1-aisoindole) conjugated to choline or a choline derivative to facilitate transport across the blood brain barrier. Methods to produce prodrugs using choline derivatives are described in United States Patent Application 2001007865. The specific examples noted in the foregoing examples are provided for illustrative purposes and are not meant in any way to limit the scope contemplated herein.

The Isoindole derivatives contemplated in the scope of this invention may be used in conjunction with one or more other drug compounds and used according to the methods of the present invention so long as the pharmaceutical agent has a use that is also effective in treating neurobehavioral disorder and/or co-morbid conditions. Pharmaceutical agents include the following categories and specific examples. It is not intended that the category be limited by the specific examples noted herein. Those of ordinary skill in the art will be able to identify readily those pharmaceutical agents that have utility with the present invention. Those of ordinary skill in the art will recognize also numerous other compounds that fall within the categories and that are useful according to the invention.
Adrenergic: Adrenalone; Amidephrine Mesylate; Apraclonidine Hydrochloride; Brimonidine Tartrate; Dapiprazole Hydrochloride; Deterenol Hydrochloride; Dipivefrin; Dopamine Hydrochloride; Ephedrine Sulfate; Epinephrine; Epinephrine Bitartrate; Epinephryl Borate; Esproquin Hydrochloride; Etafedrine Hydrochloride; Hydroxyamphetamine Hydrobromide; Levonordefrin; Mephentermine Sulfate; Metaraminol Bitartrate; Metizoline Hydrochloride; Naphazoline Hydrochloride; Norepinephrine Bitartrate; Oxidopamine; Oxymetazoline Hydrochloride; Phenylephrine Hydrochloride; Phenylpropanolamine Hydrochloride; Phenylpropanolamine Polistirex; Prenalterol Hydrochloride; Propylhexedrine; Pseudoephedrine Hydrochloride; Tetrahydrozoline Hydrochloride; Tramazoline Hydrochloride; Xylometazoline Hydrochloride.
Adrenocortical steroid: Ciprocinonide; Desoxycorticosterone Acetate; Desoxycorticosterone Pivalate; Dexamethasone Acetate; Fludrocortisone Acetate; Flumoxonide; Hydrocortisone Hemisuccinate; Methylprednisolone Hemisuccinate; Naflocort; Procinonide; Timobesone Acetate; Tipredane.
Adrenocortical suppressant: Aminoglutethimide; Trilostane.
Alcohol deterrent: Disulfiram.
Aldosterone antagonist: Canrenoate Potassium; Canrenone; Dicirenone; Mexrenoate Potassium; Prorenoate Potassium; Spironolactone.
Amino acid: Alanine; Aspartic Acid; Cysteine Hydrochloride; Cystine; Histidine; Isoleucine; Leucine; Lysine; Lysine Acetate; Lysine Hydrochloride; Methionine; Phenylalanine; Proline; Serine; Threonine; Tryptophan; Tyrosine; Valine.
Analeptic: Modafinil.
Analgesic: Acetaminophen; Alfentanil Hydrochloride; Aminobenzoate Potassium; Aminobenzoate Sodium; Anidoxime; Anileridine; Anileridine Hydrochloride; Anilopam Hydrochloride; Anirolac; Antipyrine; Aspirin; Benoxaprofen; Benzydamine Hydrochloride; Bicifadine Hydrochloride; Brifentanil Hydrochloride; Bromadoline Maleate; Bromfenac Sodium; Buprenorphine Hydrochloride; Butacetin; Butixirate; Butorphanol; Butorphanol Tartrate; Carbamazepine; Carbaspirin Calcium; Carbiphene Hydrochloride; Carfentanil Citrate; Ciprefadol Succinate; Ciramadol; Ciramadol Hydrochloride; Clonixeril; Clonixin; Codeine; Codeine Phosphate; Codeine Sulfate; Conorphone Hydrochloride; Cyclazocine; Dexoxadrol Hydrochloride; Dexpemedolac; Dezocine; Diflunisal; Dihydrocodeine Bitartrate; Dimefadane; Dipyrone; Doxpicomine Hydrochloride; Drinidene; Enadoline Hydrochloride; Epirizole; Ergotamine Tartrate; Ethoxazene Hydrochloride; Etofenamate; Eugenol; Fenoprofen; Fenoprofen Calcium; Fentanyl Citrate; Floctafenine; Flufenisal; Flunixin; Flunixin Meglumine; Flupirtine Maleate; Fluproquazone; Fluradoline Hydrochloride; Flurbiprofen; Hydromorphone Hydrochloride; Ibufenac; Indoprofen; Ketazocine; Ketorfanol; Ketorolac Tromethamine; Letimide Hydrochloride; Levomethadyl Acetate; Levomethadyl Acetate Hydrochloride; Levonantradol Hydrochloride; Levorphanol Tartrate; Lofemizole Hydrochloride; Lofentanil Oxalate; Lorcinadol; Lomoxicam; Magnesium Salicylate; Mefenamic Acid; Menabitan Hydrochloride; Meperidine Hydrochloride; Meptazinol Hydrochloride; Methadone Hydrochloride; Methadyl Acetate; Methopholine; Methotrimeprazine; Metkephamid Acetate; Mimbane Hydrochloride; Mirfentanil Hydrochloride; Molinazone; Morphine Sulfate; Moxazocine; Nabitan Hydrochloride; Nalbuphine Hydrochloride; Nalmexone Hydrochloride; Namoxyrate; Nantradol Hydrochloride; Naproxen; Naproxen Sodium; Naproxol; Nefopam Hydrochloride; Nexeridine Hydrochloride; Noracymethadol Hydrochloride; Ocfentanil Hydrochloride; Octazamide; Olvanil; Oxetorone Fumarate; Oxycodone; Oxycodone Hydrochloride; Oxycodone Terephthalate; Oxymorphone Hydrochloride; Pemedolac; Pentamorphone; Pentazocine; Pentazocine Hydrochloride; Pentazocine Lactate; Phenazopyridine Hydrochloride; Phenyramidol Hydrochloride; Picenadol Hydrochloride; Pinadoline; Pirfenidone; Piroxicam Olamine; Pravadoline Maleate; Prodilidine Hydrochloride; Profadol Hydrochloride; Propirarn Fumarate; Propoxyphene Hydrochloride; Propoxyphene Napsylate; Proxazole; Proxazole Citrate; Proxorphan Tartrate; Pyrroliphene Hydrochloride; Remifentanil Hydrochloride; Salcolex; Salethamide Maleate; Salicylamide; Salicylate Meglumine; Salsalate; Sodium Salicylate; Spiradoline Mesylate; Sufentanil; Sufentanil Citrate; Talmetacin; Talniflumate; Talosalate; Tazadolene Succinate; Tebufelone; Tetrydamine; Tifurac Sodium; Tilidine Hydrochloride; Tiopinac; Tonazocine Mesylate; Tramadol Hydrochloride; Trefentanil Hydrochloride; Trolamine; Veradoline Hydrochloride; Verilopam Hydrochloride; Volazocine; Xorphanol Mesylate; Xylazine Hydrochloride; Zenazocine Mesylate; Zomepirac Sodium; Zucapsaicin.
Anorectic compounds including dexfenfluramine.
Anorexic: Aminorex; Amphecloral; Chlorphentermine Hydrochloride; Clominorex; Clortennine Hydrochloride; Diethylpropion Hydrochloride; Fenfluramine Hydrochloride; Fenisorex; Fludorex; Fluminorex; Levamfetamine Succinate; Mefenorex Hydrochloride; Phenmetrazine Hydrochloride; Phentermine; Sibutramine Hydrochloride.
Anti-anxiety agent: Adatanserin Hydrochloride; Alpidem; Binospirone Mesylate; Bretazenil; Glemanserin; Ipsapirone Hydrochloride; Mirisetron Maleate; Ocinaplon; Ondansetron Hydrochloride; Panadiplon; Pancopride; Pazinaclone; Serazapine Hydrochloride; Tandospirone Citrate; Zalospirone Hydrochloride.
Antidepressant: Adatanserin Hydrochloride; Adinazolam; Adinazolam Mesylate; Alaproclate; Aletamine Hydrochloride; Amedalin Hydrochloride; Amitriptyline Hydrochloride; Amoxapine; Aptazapine Maleate; Azaloxan Fumarate; Azepindole; Azipramine Hydrochloride; Bipenarnol Hydrochloride; Bupropion Hydrochloride; Butacetin; Butriptyline Hydrochloride; Caroxazone; Cartazolate; Ciclazindol; Cidoxepin Hydrochloride; Cilobamine Mesylate; Clodazon Hydrochloride; Clomipramine Hydrochloride; Cotinine Fumarate; Cyclindole; Cypenamine Hydrochloride; Cyprolidol Hydrochloride; Cyproximide; Daledalin Tosylate; Dapoxetine Hydrochloride; Dazadrol Maleate; Dazepinil Hydrochloride; Desipramine Hydrochloride; Dexamisole; Deximafen; Dibenzepin Hydrochloride; Dioxadrol Hydrochloride; Dothiepin Hydrochloride; Doxepin Hydrochloride; Duloxetine Hydrochloride; Eclanamine Maleate; Encyprate; Etoperidone Hydrochloride; Fantridone Hydrochloride; Fehmetozole Hydrochloride; Fenmetramide; Fezolamine Fumarate; Fluotracen Hydrochloride; Fluoxetine; Fluoxetine Hydrochloride; Fluparoxan Hydrochloride; Gamfexine; Guanoxyfen Sulfate; Imafen Hydrochloride; Imiloxan Hydrochloride; Imipramine Hydrochloride; Indeloxazine Hydrochloride; Intriptyline Hydrochloride; Iprindole; Isocarboxazid; Ketipramine Fumarate; Lofepramine Hydrochloride; Lortalamine; Maprotiline; Maprotiline Hydrochloride; Melitracen Hydrochloride; Milacemide Hydrochloride; Minaprine Hydrochloride; Mirtazapine; Moclobemide; Modaline Sulfate; Napactadine Hydrochloride; Napamezole Hydrochloride; Nefazodone Hydrochloride; Nisoxetine; Nitrafudam Hydrochloride; Nomifensine Maleate; Nortriptyline Hydrochloride; Octriptyline Phosphate; Opipramol Hydrochloride; Oxaprotiline Hydrochloride; Oxypertine; Paroxetine; Phenelzine Sulfate; Pirandamine Hydrochloride; Pizotyline; Pridefine Hydrochloride; Prolintane Hydrochloride; Protriptyline Hydrochloride; Quipazine Maleate; Rolicyprine; Seproxetine Hydrochloride; Sertraline Hydrochloride; Sibutramine Hydrochloride; Sulpiride; Suritozole; Tametraline Hydrochloride; Tampramine Fumarate; Tandamine Hydrochloride; Thiazesim Hydrochloride; Thozalinone; Tomoxetine Hydrochloride; Trazodone Hydrochloride; Trebenzomine Hydrochloride; Trimipramine; Trimipramine Maleate; Venlafaxine Hydrochloride; Viloxazine Hydrochloride; Zimeldine Hydrochloride; Zometapine.
Antihypertensive: Aflyzosin Hydrochloride; Alipamide; Althiazide; Amiquinsin Hydrochloride; Amlodipine Besylate; Amlodipine Maleate; Anaritide Acetate; Atiprosin Maleate; Belfosdil; Bemitradine; Bendacalol Mesylate; Bendroflumethiazide; Benzthiazide; Betaxolol Hydrochloride; Bethanidine Sulfate; Bevantolol Hydrochloride; Biclodil Hydrochloride; Bisoprolol; Bisoprolol Fumarate; Bucindolol Hydrochloride; Bupicomide; Buthiazide: Candoxatril; Candoxatrilat; Captopril; Carvedilol; Ceronapril; Chlorothiazide Sodium; Cicletanine; Cilazapril; Clonidine; Clonidine Hydrochloride; Clopamide; Cyclopenthiazide; Cyclothiazide; Darodipine; Debrisoquin Sulfate; Delapril Hydrochloride; Diapamide; Diazoxide; Dilevalol Hydrochloride; Diltiazem Malate; Ditekiren; Doxazosin Mesylate; Ecadotril; Enalapril Maleate; Enalaprilat; Enalkiren; Endralazine Mesylate; Epithiazide; Eprosartan; Eprosartan Mesylate; Fenoldopam Mesylate; Flavodilol Maleate; Flordipine; Flosequinan; Fosinopril Sodium; Fosinoprilat; Guanabenz; Guanabenz Acetate; Guanacline Sulfate; Guanadrel Sulfate; Guancydine; Guanethidine Monosulfate; Guanethidine Sulfate; Guanfacine Hydrochloride; Guanisoquin Sulfate; Guanoclor Sulfate; Guanoctine Hydrochloride; Guanoxabenz; Guanoxan Sulfate; Guanoxyfen Sulfate; Hydralazine Hydrochloride; Hydralazine Polistirex; Hydroflumethiazide; Indacrinone; Indapamide; Indolaprif Hydrochloride; Indoramin; Indoramin Hydrochloride; Indorenate Hydrochloride; Lacidipine; Leniquinsin; Levcromakalim; Lisinopril; Lofexidine Hydrochloride; Losartan Potassium; Losulazine Hydrochloride; Mebutamate; Mecamylamine Hydrochloride; Medroxalol; Medroxalol Hydrochloride; Methalthiazide; Methyclothiazide; Methyldopa; Methyldopate Hydrochloride; Metipranolol; Metolazone; Metoprolol Fumarate; Metoprolol Succinate; Metyrosine; Minoxidil ; Monatepil Maleate; Muzolimine; Nebivolol; Nitrendipine; Ofornine; Pargyline Hydrochloride; Pazoxide; Pelanserin Hydrochloride; Perindopril Erbumine; Phenoxybenzamine Hydrochloride; Pinacidil; Pivopril; Polythiazide; Prazosin Hydrochloride; Primidolol; Prizidilol Hydrochloride; Quinapril Hydrochloride; Quinaprilat; Quinazosin Hydrochloride; Quinelorane Hydrochloride; Quinpirole Hydrochloride; Quinuclium Bromide; Ramipril; Rauwolfia Serpentina; Reserpine; Saprisartan Potassium; Saralasin Acetate; Sodium Nitroprusside; Sulfinalol Hydrochloride; Tasosartan; Teludipine Hydrochloride; Temocapril Hydrochloride; Terazosin Hydrochloride; Terlakiren; Tiamenidine; Tiamenidine Hydrochloride; Ticrynafen; Tinabinol; Tiodazosin; Tipentosin Hydrochloride; Trichlormethiazide; Trimazosin Hydrochloride; Trimethaphan Camsylate; Trimoxamine Hydrochloride; Tripamide; Xipamide; Zankiren Hydrochloride; Zofenoprilat Arginine.
Anti-inflammatory: Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Momiflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Zomepirac Sodium.
Antinauseant: Buclizine Hydrochloride; Cyclizine Lactate; Naboctate Hydrochloride.
Antineutropenic: Filgrastim; Lenograstim; Molgramostim; Regramostim; Sargramostim.
Antiobsessional agent: Fluvoxamine Maleate.
Antiparkinsonian: Benztropine Mesylate; Biperiden; Biperiden Hydrochloride; Biperiden Lactate; Carmantadine; Ciladopa Hydrochloride; Dopamantine; Ethopropazine Hydrochloride; Lazabemide; Levodopa; Lometraline Hydrochloride; Mofegiline Hydrochloride; Naxagolide Hydrochloride; Pareptide Sulfate; Procyclidine Hydrochloride; Quinetorane Hydrochloride; Ropinirole Hydrochloride; Selegiline Hydrochloride; Tolcapone; Trihexyphenidyl Hydrochloride. Antiperistaltic: Difenoximide Hydrochloride; Difenoxin; Diphenoxylate Hydrochloride; Fluperamide; Lidamidine Hydrochloride; Loperamide Hydrochloride; Malethamer; Nufenoxole; Paregoric.
Antipsychotic: Acetophenazine Maleate; Alentemol Hydrobromide; Alpertine; Azaperone; Batelapine Maleate; Benperidol; Benzindopyrine Hydrochloride; Brofbxine; Bromperidol; Bromperidol Decanoate; Butaclamol Hydrochloride; Butaperazine; Butaperazine Maleate; Carphenazine Maleate; Carvotroline Hydrochloride; Chlorpromazine; Chlorpromazine Hydrochloride; Chlorprothixene; Cinperene; Cintriamide; Clomacran Phosphate; Clopenthixol; Clopimozide; Clopipazan Mesylate; Cloroperone Hydrochloride; Clothiapine; Clothixamide Maleate; Clozapine; Cyclophenazine Hydrochloride; Droperidol; Etazolate Hydrochloride; Fenimide; Flucindole; Flumezapine; Fluphenazine Decanoate; Fluphenazine Enanthate; Fluphenazine Hydrochloride; Fluspiperone; Fluspirilene; Flutroline; Gevotroline Hydrochloride; Halopemide; Haloperidol; Haloperidol Decanoate; Iloperidone; Imidoline Hydrochloride; Lenperone; Mazapertine Succinate; Mesoridazine; Mesoridazine Besylate; Metiapine; Milenperone; Milipertine; Molindone Hydrochloride; Naranol Hydrochloride; Neflumozide Hydrochloride; Ocaperidone; Olanzapine; Oxiperomide; Penfluridol; Pentiapine Maleate; Perphenazine; Pimozide; Pinoxepin Hydrochloride; Pipamperone; Piperacetazine; Pipotiazine Palniitate; Piquindone Hydrochloride; Prochlorperazine Edisylate; Prochlorperazine Maleate; Promazine Hydrochloride; Remoxipride; Remoxipride Hydrochloride; Rimcazole Hydrochloride; Seperidol Hydrochloride; Sertindole; Setoperone; Spiperone; Thioridazine; Thioridazine Hydrochloride; Thiothixene; Thiothixene Hydrochloride; Tioperidone Hydrochloride; Tiospirone Hydrochloride; Trifluoperazine Hydrochloride; Trifluperidol; Triflupromazine; Triflupromazine Hydrochloride; Ziprasidone Hydrochloride.
Appetite suppressant: Dexfenfluramine Hydrochloride; Phendimetrazine Tartrate; Phentermine Hydrochloride.
Blood glucose regulators: Human insulin; Glucagon; Tolazamide; Tolbutamide; Chloropropamide; Acetohexamide and Glipizide.
Carbonic anhydrase inhibitor: Acetazolamide; Acetazolamide Sodium, Dichlorphenamide; Dorzolamide Hydrochloride; Methazolamide; Sezolarmide Hydrochloride.
Cardiac depressant: Acecainide Hydrochloride; Acetylcholine Chloride; Actisomide; Adenosine; Amiodarone; Aprindine; Aprindine Hydrochloride; Artilide Fumarate; Azimilide Dihydrochloride; Bidisomide; Bucainide Maleate; Bucromarone; Butoprozine Hydrochloride; Capobenate Sodium; Capobenic Acid; Cifenline; Cifenline Succinate; Clofilium Phosphate; Disobutamide; Disopyramide; Disopyramide Phosphate; Dofetilide; Drobuline; Edifolone Acetate; Emilium Tosylate; Encainide Hydrochloride; Flecainide Acetate; Ibutilide Fumarate; Indecainide Hydrochloride; Ipazilide Fumarate; Lorajmine Hydrochloride; Lorcainide Hydrochloride; Meobentine Sulfate; Mexiletine Hydrochloride; Modecainide; Moricizine; Oxiramide; Pirmenol Hydrochloride; Pirolazamide; Pranolium Chloride; Procainamide Hydrochloride; Propafenone Hydrochloride; Pyrinoline; Quindonium Bromide; Quinidine Gluconate; Quinidine Sulfate; Recainam Hydrochloride; Recainam Tosylate; Risotilide Hydrochloride; Ropitoin Hydrochloride; Sematilide Hydrochloride; Suricainide Maleate; Tocainide; Tocainide Hydrochloride; Transcainide.
Cardiotonic: Actodigin; Amrinone; Bemoradan; Butopamine; Carbazeran; Carsatrin Succinate; Deslanoside; Digitalis; Digitoxin; Digoxin; Dobutamine; Dobutamine Hydrochloride; Dobutamine Lactobionate; Dobutamine Tartrate; Enoximone; Imazodan Hydrochloride; Indolidan; Isomazole Hydrochloride; Levdobutamine Lactobionate; Lixazinone Sulfate; Medorinone; Milrinone; Pelrinone Hydrochloride; Pimobendan; Piroximone; Prinoxodan; Proscillaridin; Quazinone; Tazolol Hydrochloride; Vesnarinone.
Cardiovascular agent: Dopexamine; Dopexamine Hydrochloride.
Choleretic: Dehydrocholic Acid; Fencibutirol; Hymecromone; Piprozolin; Sincalide; Tocamphyl.
Cholinergic: Aceclidine; Bethanechol Chloride; Carbachol; Demecarium Bromide; Dexpanthenol; Echothiophate Iodide; Isoflurophate; Methacholine Chloride; Neostigmine Bromide; Neostigmine Methylsulfate; Physostigmine; Physostigmine Salicylate; Physostigmine Sulfate; Pilocarpine; Pilocarpine Hydrochloride; Pilocarpine Nitrate; Pyridostigmine Bromide.
Cholinergic agonist: Xanomeline; Xanomeline Tartrate.
Cholinesterase Deactivator: Obidoxime Chloride; Pralidoxime Chloride; Pralidoxime Iodide; Pralidoxime Mesylate.
Coccidiostat: Arprinocid; Narasin; Semduramicin; Semduramicin Sodium.
Cognition adjuvant: Ergoloid Mesylates; Piracetam; Pramiracetam Hydrochloride; Pramiracetam Sulfate; Tacrine Hydrochloride.
Cognition enhancer: Besipirdine Hydrochloride; Linopirdine; Sibopirdine.
Hormone: Diethylstilbestrol; Progesterone; 17 hydroxy progesterone; Medroxyprogesterone; Norgestrel; Norethynodrel; Estradiol; Megestrol (Megace); Norethindrone; Levonorgestrel; Ethyndiol; Ethinyl estradiol; Mestranol; Estrone; Equilin; 17 alpha dihydroequilin; equilenin; 17 alpha dihydroequilenin; 17 alpha estradiol; 17 beta estradiol; Leuprolide (lupron); Glucagon; Testolactone; Clomiphene; Han memopausal gonadotropins; Human chorionic gonadotropin; Urofollitropin; Bromocriptine; Gonadorelin; Luteinizing hormone releasing hormone and analogs; Gonadotropins; Danazol; Testosterone; Dehydroepiandrosterone; Androstenedione; Dihydroestosterone; Relaxin; Oxytocin; Vasopressin; Folliculostatin; Follicle regulatory protein; Gonadoctrinins; Oocyte maturation inhibitor; Insulin growth factor; Follicle Stimulating Hormone; Luteinizing hormone; Tamoxifen.; Corticorelin Ovine Triftutate; Cosyntropin; Metogest; Pituitary, Posterior; Seractide Acetate; Somalapor; Somatrem; Somatropin; Somenopor; Somidobove.
Memory adjuvant: Dimoxamine Hydrochloride; Ribaminol.
Mental performance enhancer: Aniracetam.
Mood regulator: Fengabine.
Neuroleptic: Duoperone Fumarate; Risperidone.
Neuroprotective: Dizocilpine Maleate.
Psychotropic: Minaprine.
Relaxant: Adiphenine Hydrochloride; Alcuronium Chloride; Aminophylline; Azumolene Sodium; Baclofen; Benzoctamine Hydrochloride; Carisoprodol; Chlorphenesin Carbamate; Chlorzoxazone; Cinflumide; Cinnamedrine; Clodanolene; Cyclobenzaprine Hydrochloride; Dantrolene; Dantrolene Sodium; Fenalanide; Fenyripol Hydrochloride; Fetoxylate Hydrochloride; Flavoxate Hydrochloride; Fletazepam; Flumetramide;-Flurazepam Hydrochloride; Hexafluorenium Bromide; Isomylamine Hydrochloride; Lorbamate; Mebeverine Hydrochloride; Mesuprine Hydrochloride; Metaxalone; Methocarbamol; Methixene Hydrochloride; Nafomine Malate; Nelezaprine Maleate; Papaverine Hydrochloride; Pipoxolan Hydrochloride; Quinctolate; Ritodrine; Ritodrine Hydrochloride; Rolodine; Theophylline Sodium Glycinate; Thiphenamil Hydrochloride; Xilobam.
Sedative-hypnotic: Allobarbital; Alonimid; Alprazolam; Amobarbital Sodium; Bentazepam; Brotizolam; Butabarbital; Butabarbital Sodium; Butalbital; Capuride; Carbocloral; Chloral Betaine; Chloral Hydrate; Chlordiazepoxide Hydrochloride; Cloperidone Hydrochloride; Clorethate; Cyprazepam; Dexclamol Hydrochloride; Diazepam; Dichloralphenazone; Estazolam; Ethchlorvynol; Etomidate; Fenobam; Flunitrazepam; Fosazepam; Glutethimide; Halazepam; Lormetazepam; Mecloqualone; Meprobamate; Methaqualone; Midaflur; Paraldehyde; Pentobarbital; Pentobarbital Sodium; Perlapine; Prazepam; Quazepam; Reclazepam; Roletamide; Secobarbital; Secobarbital Sodium; Suproclone; Thalidomide; Tracazolate; Trepipam Maleate; Triazolam; Tricetamide; Triclofos Sodium; Trimetozine; Uldazepam; Zaleplon; Zolazepam Hydrochloride; Zolpidem Tartrate.
Serotonin antagonist: Altanserin Tartrate; Amesergide; Ketanserin; Ritanserin.
Serotonin inhibitor: Cinanserin Hydrochloride; Fenclonine; Fonazine Mesylate; Xylamidine Tosylate.
Serotonin receptor antagonist: Tropanserin Hydrochloride.
Stimulant: Amfonelic Acid; Amphetamine Sulfate; Ampyzine Sulfate; Arbutamine Hydrochloride; Azabon; Caffeine; Ceruletide; Ceruletide Diethylamine; Cisapride; Dazopride Fumarate; Dextroamphetamine; Dextroamphetamine Sulfate; Difluanine Hydrochloride; Dimefline Hydrochloride; Doxapram Hydrochloride; Etryptamine Acetate; Ethamivan; Fenethylline Hydrochloride; Flubanilate Hydrochloride; Flurothyl; Histamine Phosphate; Indriline Hydrochloride; Mefexamide; Methamphetamine Hydrochlo ride; Methylphenidate Hydrochloride; Pemoline; Pyrovalerone Hydrochloride; Xamoterol; Xamoterol Fumarate. Synergist: Proadifen Hydrochloride.
Thyroid hormone: Levothyroxine Sodium; Liothyronine Sodium; Liotrix.
Thyroid inhibitor: Methimazole; Propyithiouracil.
Thyromimetic: Thyromedan Hydrochloride.
Cerebral ischemia agents: Dextrorphan Hydrochloride.
Vasoconstrictor: Angiotensin Amide; Felypressin; Methysergide; Methysergide Maleate.
Vasodilator: Alprostadil; Azaclorzine Hydrochloride; Bamethan Sulfate; Bepridil Hydrochloride; Buterizine; Cetiedil Citrate; Chromonar Hydrochloride; Clonitrate; Diltiazem Hydrochloride; Dipyridamole; Droprenilamine; Erythrityl Tetranitrate; Felodipine; Flunarizine Hydrochloride; Fostedil; Hexobendine; Inositol Niacinate; Iproxamine Hydrochloride; Isosorbide Dinitrate; Isosorbide Mononitrate; Isoxsuprine Hydrochloride; Lidoflazine; Mefenidil; Mefenidil Fumarate; Mibefradil Dihydrochloride; Mioflazine Hydrochloride; Mixidine; Nafronyl Oxalate; Nicardipine Hydrochloride; Nicergoline; Nicorandil; Nicotinyl Alcohol; Nifedipine; Nimodipine; Nisoldipine; Oxfenicine; Oxprenolol Hydrochloride; Pentaerythritol Tetranitrate; Pentoxifylline; Pentrinitrol; Perhexiline Maleate; Pindolol; Pirsidomine; Prenylamine; Propatyl Nitrate; Suloctidil; Terodiline Hydrochloride; Tipropidil Hydrochloride; Tolazoline Hydrochloride; Xanthinol Niacinate.

When administered, the formulations of the invention are applied in pharmaceutically acceptable amounts and in pharmaceutically acceptable compositions. Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulfuric, nitric, phosphoric, maleic, acetic, salicylic, p-toluene sulfonic, tartaric, citric, methane sulfonic, formic, malonic, succinic, naphthalene-2-sulfonic, and benzene sulfonic. Also, pharmaceutically acceptable salts can be prepared as alkaline metal or alkaline earth salts, such as sodium, potassium or calcium salts.

Suitable buffering agents include: acetic acid and a salt (1-2% W/V); citric acid and a salt (1-3% W/V); boric acid and a salt (0.5-2.5% W/V); and phosphoric acid and a salt (0.8-2% W/V). Suitable preservatives include benzalkonium chloride (0.003-0.03% W/V); chlorobutanol (0.3-0.9% W/V); parabens (0.01-0.25% W/V) and thimerosal (0.004-0.02% W/V).

In the present invention, the Isoindole derivatives according to formula (I) are administered in safe and effective amounts. An effective amount means that amount necessary to delay; the onset, inhibit the progression, halt altogether the onset or progression of, or to reduce the clinical manifestations or symptoms of the particular condition being treated. In general, an effective amount for treating a neurobehavioral disorder will be that amount necessary to inhibit the symptoms of the particular neurobehavioral disorder in-situ in a particular individual. When administered to an individual, effective amounts will depend, of course, on the particular condition being treated; the severity of the condition; individual patient parameters including age, physical condition, size and weight; concurrent treatment; frequency of treatment; and the mode of administration. These factors are well known to those of ordinary skill in the art and can be addressed with no more than routine experimentation. It is preferred generally that a minimum dose be used, that is, the lowest safe dosage that provides appropriate relief of symptoms.

Dosage may be adjusted appropriately to achieve desired drug levels, locally or systemically. Generally, daily doses of active compounds will be from about 0.001 mg/kg per day to 200 mg/kg per day. However, it is recognized that these are general ranges and the actual dose used as contemplated in a given individual may less or greater than this dosage range. In the event that the response in an individual subject is insufficient at such doses, even higher doses (or effective higher doses by a different, more localized delivery route) may be employed to the extent that patient tolerance permits.

A variety of administration routes are available. The particular mode selected will depend of course, upon the particular drug selected, the severity of the disease state(s) being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects and multiple doses over a given period of time are also contemplated. Such modes of administration include oral, rectal, sublingual, topical, nasal, transdermal or parenteral routes. The term "parenteral" includes subcutaneous, intravenous, intramuscular, or infusion. Depot intramuscular injections suitably prepared may also be used for administration within the scope of this invention.

The compositions may conveniently be presented in unit dosage form and may be prepared by any of the methods well known in the art of pharmacy. In general, the compositions are prepared by uniformly and intimately bringing the compounds into association with a liquid carrier, a finely divided solid carrier, or both, and then, if necessary, shaping the product.

Compositions suitable for oral administration may be presented as discrete units such as capsules, cachets, tablets, or lozenges, each containing a predetermined amount of the active compound. Other compositions include suspensions in aqueous liquors or non-aqueous liquids such as; a syrup, an elixir, or an emulsion.

Other delivery systems can include time-release, delayed release or sustained release delivery systems. Such systems can avoid repeated administrations of the active compounds of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. In addition, a pump-based hardware delivery system can be used, some of which are adapted for implantation.

Long-term sustained release devices, pharmaceutical compositions or molecular derivatives also may be used with the Isoindole derrivatives described by the inventors in the current invention. "Long-term" release, as used herein, means that the drug delivery devise is constructed and arranged to deliver therapeutic levels of the active ingredient for at least 2 days, and preferably as long as 60 days. Long-term sustained release devices such as patches, implants and suppositories are well known to those of ordinary skill in the art and include some of the release systems described above. It is also contemplated by the inventors that the Isoindole derivatives described by the inventors may be formulated in such ways as to achieve various plasma profiles of the Isoindole derivatives in given individuals so as to maintain certain effective profiles of given plasma levels over a period of time. Such formulation strategies are well known to those skilled in the art and may for example include special coatings on tablets or granules containing the Isoindole derivatives of this invention either alone or in combination with other pharmacologically active substances. All such formulations are contemplated with the scope of this invention.

### EXPERIMENTAL

The involvement of the TMMS in a wide range of otherwise unrelated neurobehavioral conditions and the ability of the compounds of formula I to treat these conditions though balanced trimonoamine uptake inhibition is based on the results of the experiments and clinical case studies below. These examples are offered by way of illustration and are not intended to limit the invention in any manner.
Example 1: To determine if all three monoamine transporters of the TMMS were involved with the pathophysiology of various neurobehavioral disorders, polymorphic variants within genes regulating synthesis, metabolism, and receptor binding of DA, 5HT and NE were examined. Genomic deoxyribonucleic acid (DNA) was obtained from patient's with various neurobehavioral disorders selected from the categories of: 1) pervasive developmental disorders and/or autism spectrum disorders (PNDS), 2) impulse control disorders and/or reward deficiency disorders (RDDS), 3) obsessive-compulsive spectrum disorders (OCDS), 4) dementia disorders (DEMS), 5) somatoform disorders (SOMS) and 6) movement disorders (MOVS). To examine the pharmacologic targets of the compounds that are the subject of this patent application, polymorphic variants present in the genomic DNA of patients afflicted with the disorders were compared to those in the DNA of non-affected control individuals. The Solute Carrier (SLC) genes; SLC6A3, SLC6A4 and SLC6A2 which encode for the Dopamine transporter (DAT) protein, Serotonin transporter (SERT) protein, and Norepinephrine transporter (NET) protein respectively, were examined. In these studies, three polymorphic variants in the SLC6A3 gene: DAT1 (Vandenberg DJ, et al., 2000), DAT2 (Rubie C, et al., 2001) DAT3 (Vandenberg DJ, et al., 1992), and three polymorphic variants in the SLC6A4 gene SERT1 (Battersby S, et al., 1999), SERT2 (Heils A, et al., 1996), SERT3 (Ogilive AD, et al., 1996), as well as three polymorphic variants in the SLC6A2 gene: NET1 (Urwin RE, et al. 2002), NET2 (Kim CH, et al., 2006) NET3 (Stober G, et al., 1996) were studied using published techniques. Statistical analysis was performed using Person's Chi square and logistic regression analysis.
   The results (Table 1) of analysis revealed that specific polymorphic genetic variants in all three monoamine transporters of the TMMS system were found preferentially in human individuals suffering from these neurobehavioral disorders rather than in control human individuals not afflicted with the disorders. This data indicates that alterations of the TMMS and imbalance of the trimonoamines therein, associated with functional variation of the trimonoamine transporters underlies all these disorders.
Example 2: Several compounds according to Formula 1 were tested in vitro for their ability to simultaneously and differentially inhibit binding of radioligands to dopamine (DA), norepinephrine (NE) and serotonin (5HT) transporters in the desired Ki ratio. These transporters control the amount of the three monoamines in the TMMS and therefore regulate fast excitatory and inhibitory neurotransmission in the CNS. Thus, the relative amounts of all three of these monoamines in the TMMS of brain controls mammalian neurobehaviors. To determine the Ki of the representative compounds; recombinant human monoamine transporters (DAT, SERT and NET) were expressed in HEK-293 cells as described in Eshleman et al. (1999). The HEK-hDAT, HEK-hSERT and HEK-hNET cells were incubated in modified Eagle's medium or Dulbecco's modified Eagle's medium exactly as described by Eshleman et al. Cells were grown until confluent on 150 mm diameter tissue culture dishes in a humidified 10% CO2 environment at 37°C. The Ki values of Mazindol, compound 1, 2 and 3 for recombinant human DAT, NET, and SERT expressed in HEK-293 cells was determined in competition binding assays by the inhibition of : [3supH] Paroxetine (SERT), [125sup I] RTI-55 binding (DAT) and (NET). The binding assays contained an aliquot of membranes prepared from the HEK cell lines expressing the recombinant human transporters (≈12-30 µg protein, depending on the cell line, adjusted to bind <10% of the total radioactivity added to the assay), [125supl] RTI-55 (0.2 nM final concentration) or [3supH] Paroxetine (0.4 nM final concentration) along with Krebs-HEPES assay buffer to yield a final volume of 250 µL. Specific binding was defined as the difference in binding observed in the presence and absence of 10 µM nomifensine (HEK-hDAT), 10 uM desipramine HEK-NET) or 10 µM imipramine (HEK-hSERT). The reaction was incubated for 90 min at room temperature in the dark and was terminated by vacuum filtration. Competition experiments were conducted with duplicate determinations.
   Table 2 indicates that compounds according to Formula 1 and the exemplary molecule Mazindol simultaneously binds to all three of these monoamine transporters and have a relatively equal inhibitory effect. Therefore they have pharmacologic activity on the TMMS for the range of neurobehavioral disorders associated with these polymorphic variants. Notably, each compound may have a differential, but yet nearly equal effect on the DA, 5HT and NE transporters and thus simultaneously regulate the amount of DA, 5HT and NE available. Thus the compounds according to formula (I) are "balanced trimonoamine uptake inhibitors"
Example 3: P. M. is a 14 year old male. At 4 years of age a diagnosis of ADHD was made and he was treated with Ritalin (methylphenidate) with some improvement in attention span but little effect on several other neurobehavioral symptoms, including abusive behavior and depression. At age 9 years of age, he was also being treated with risperidone which continued for 2 years. At 13 years of age the patient was started on Mazindol at 2.5 mg BID and the Ritalin was discontinued. Within 2 months he reported improved attention span, loss of hyperactivity and his bad behavior as a result of his impulsivity significantly improved. Subsequently, the risperidone was then completely discontinued without any recurrence of symptoms of ADHD (i.e. poor attention span, hyperactivity, or inappropriate behavior). The patient was then maintained on Mazindol at 2.5 mg/day as the sole agent without the return of any symptoms of ADHD over the next 3 years.
Example 4: J. K. is a 16 year old male. He began to have constant vocal tics at age 3. Motor tics started at age 4, consisting of licking his lips, rolling his eyes, hand and foot tics. He was diagnosed as having Tourette syndrome. At 6 years of age a diagnosis of ADHD was also made and he was treated with methyphenidate with some improvement in hyperactivity but little effect on his inability to focus and concentrate. At age 8, the major problems were with his tics, outbursts and lack of control. At 10 years of age, despite treatment with methylphenidate 20 mg twice a day (bid), haloperidol 0.5 mg and paroxetine 10 mg, he was in special school and was suffering from uncountable motor and vocal tics everyday during waking hours and with outbursts of shouting, coprolalia and episodes of self-mutilation. coprolabia, lying, hitting things and people, and extremely oppositional. Treatment with Mazindol 7.5 mg per day was begun. This led to significant improvement in these symptoms and within 4 months the paroxetine and methylphenidate were discontinued, and he was then maintained on Mazindol and low dose risperidone with excellent clinical effect.
Example 5: P.C. a 48 year old female had suffered from chronic fatigue syndrome, "chemical sensitivity and for one year with marked fatigue each day beginning in the early or mid afternoon. There has been some accompanying muscle aching, but no symptoms of central neurosystem involvement. Her functioning at work and at home had been seriously impaired. EBV titers were positive. She was started on Mazindol 5.0 mg per day. Subsequently, she experienced complete relief of her fatigue and muscle ache symptoms after 10 days of therapy, and this improvement was sustained thereafter on a 5 mg per day dose.
Example 6: W. K. a 45 year old male had suffered from alcohol abuse, sexual addiction and pathologic gambling for 10 years. He had been in counseling, Alcoholic Anonymous, Gamblers Anonymous on and off over the past 10 years with minimal effects. He had lost his marriage and several jobs as result of his behavioral problems. He had been treated with antidepressants, but they had been of little help. He was diagnosed with symptoms of a reward deficiency disorder and was then treated with Mazindol 2 mg BID. Over the next several weeks he reported a loss or his desire to drink and gamble was able to completely stop drinking after 5 weeks for the fist time in 10 years despite having been in Alcoholics Anonymous. In addition, he reported that the thrill he has always gotten from gambling was no long present and he no longer felt compulsion to gamble. The improvement in behavior continued for the entire time he was taking Mazindol.
Example 7: R O is a 68 year old male had suffered from motor impairment and cognitive decline since age 60. By age 66 he developed frank dementia and required institutionalization and nursing care. He was started on Mazindol at 8 mg per day and there was improvement of his motor function, and his dementia after 2 weeks of treatment. Based on reports by his care givers his became more aware of surrounding and was able to recognize family member for the first time in one year and was not "hearing and seeing things that were not there".
Example 8: L. P is a 32 year old female who developed compulsive behaviors after the birth of her first child. She became obsessed with the thought of "bacteria contaminating everything", this worsen when she had a second child. She washed all food even if it had been pre-packaged and placed in specific foods in specific locations in her pantry and refrigerator which she was sure were the "safest: She wore gloves when she left the house and made her children do so as well. She sprayed disinfectant on everything she or her children came in contact with and refused in eat in restaurants. Prior to her pregnancy she had been diagnosed with polycystic ovary disease and need treatment in order to conceive. She was told that she was also "borderline diabetic". She began treatment with Mazindol 5 mg per day. Shortly, after beginning her therapy she reported that "mood was better" and she didn't "feel anxious" about bacteria anymore. After three weeks of therapy with Mazindol she stopped wearing gloves (and forcing her children to do so) and using disinfectant. Her husband reported that she was a "new woman" and he was no allowed to put the groceries away without having to place it in particular places. The relief of her obsessive compulsive behavior continued as long as she was taking Mazindol.

While the present invention has been disclosed in this patent application by reference to details of preferred embodiments of the invention, it is to be understood that the disclosure is intended to be illustrative rather than in a limiting sense. It is contemplated that many alternatives, modifications and variations thereof will be apparent to those of ordinary skill in the art. All such alternatives, modifications, and variations are intended to fall within the spirit of the present invention and the scope of the appended claims.

### REFERENCES

L.W. Swanson and G.D. Petrovich. What is the amygdala? Trends Neurosci. 21; 23-331. (1998)
L. Heimer, A new anatomical framework for neuropsychiatric disorders and drug abuse, Am. J. Psychiatr. 160; 1726-1739. (2003)
P.W. Kalivas and C.D. Barnes, Limbic motor circuits and neuropsychiatry, CRC Press, Boca Raton (1993).
van Groen T. Kadish I. Wyss JM. Species differences in the projections from the entorhinal cortex to the hippocampus. Brain Research Bulletin. 57(3-4):553-6, (2002)
Heidbreder CA. Groenewegen HJ. The medial prefrontal cortex in the rat: evidence for a dorso-ventral distinction based upon functional and anatomical characteristics. Neuroscience & Biobehavioral Reviews. 27(6):555-79, (2003).
Jones BJ, Blackburn TP. The medical benefit of 5-HT research. Pharmacol Biochem Behav Apr;71(4):555-681 (2002)
Othmer E. Othmer JP. Othmer SC. Brain functions and psychiatric disorders. A clinical view. Psychiatric Clinics of North America. 21(3):517-66, (1998) Sep.
American Psychiatric Association. Diagnostic and statistical manual of mental disorders: DSM-IV-TR 4th ed. text version. Washington DC: APA; 2000
Griggs RC. Moxley RT. Mendell JR., et al. Randomized, double-blind trial of mazindol in Duchenne dystrophy. Muscle Nerve. 13(12):1169-73 (1990) Dec
Alvarez B, Dahlitz M, Grimshaw J, Parkes JD. Mazindol in long-term treatment of narcolepsy. Lancet. 25; 337(8752):1293-4. (1991) May
Woodhouse CR, Tiptaft RC. Mazindol in the control of micturition. Br J Urol. Dec;55(6):636-8. (1983)
Cheng Y, Prusoff WH. Relationship between the inhibition constant ([Ki]) and the concentration of inhibitor which causes 50 per cent inhibition ([IC50]) of an enzymatic reaction. Biochem Pharm 22:3099-3108 (1973).
Green et al., "Protective Groups in Organic Chemistry". John Wiley & Sons, Inc., 2nd ed. New York, NY (1991)
Kramer, W., Bile acid derived HMG-CoA reductase inhibitors, Biochim. Biophys. Acta. 1227: 137-154 (1994b).
Vandenbergh DJ, Persico AM, Hawkins AL, at al. Human dopamine transporter gene (DAT1) maps to chromosome 5p15.3 and displays a VNTR. Genomics 14: 1104-1106 (1992)
Rubie C, Schmidt F, Knapp M, et al. The human dopamine transporter gene: the 5' - flanking region reveals five diallelic polymorphic sites in a Caucasian population sample. Neurosci Lett 297; 125-128 (2001)
Vandenbergh DJ, Thompson MD, Cook EH, et al. Human dopamine transporter gene: coding region conservation among normal, Tourette's disorder, alcohol dependence and attention-deficit hyperactivity disorder populations. Mol Psychiatry 2; 283-292 (2000)
Heils A, Teufel A, Petri S, et al. Allelic Variation of Human Serotonin Transporter Gene Expression. J Neurochem. 66(6):2621-2624 (1996)
Ogilvie AD, Battersby S, Bubb V, et al. Polymorphism in serotonin transporter gene associated with susceptibility to major depression. Lancet. 347(9003):731-733 (March 16, 1996)
Battersby S, Ogilvie AD, Blackwood DH, et al. Presence of multiple functional polyadenylation signals and a single nucleotide polymorphism in the 3' untranslated region of the human serotonin transporter gene. J Neurochem. 72(4):1384-1388 (1999)
Urwin RE, Bennetts B, Wilcken B, et al. Anorexia nervosa (restrictive subtype) is associated with a polymorphism in the novel norepinephrine transporter gene promoter polymorphic region. Molecular Psychiatry. 7(6):652-657 (2002)
Kim CH, Hahn MK, Joung Y, et al. A polymorphism in the norepinephrine transporter gene alters promoter activity and is associated with attention-deficit hyperactivity disorder. Proc Natl Acad Sci USA. 103(50):19164-19169 (2006)
Stober G, Nothen MM, Porzgen P, et al. Systematic search for variation in the human norepinephrine transporter gene: identification of five naturally occurring missense mutations and study of association with major psychiatric disorders. Am J Med Genet. 67(6):523-532 (1996)
Eshleman, A.J., Carmolli, M., et al. Characteristics of drug interactions with recombinant biogenic amine transorters expressed in the same cell type. Journal of Pharmacology and Experimental Therapeutics 289: 877-885 (1999).

**Table 1 SLC6A3, SLC6A4 and SLC6A2 genotypes and neurobehavioral disorders**

| Locus | Disorders | Number | Genotype1 | Genotype2 | Genotype3 | Chi Sq. | P value |
|---|---|---|---|---|---|---|---|
| DAT1 | control | 294 | 20 | 114 | 160 | ----- | ----- |
| DAT1 | PNDS | 170 | 21 | 73 | 76 | 6.21 | 0.04 |
| DAT1 | RDDS | 161 | 14 | 66 | 81 | 0.96 | NS |
| DAT1 | OCDS | 131 | 9 | 72 | 50 | 10.27 | 0.0059 |
| DAT1 | DEMS | 148 | 9 | 58 | 81 | 0.08 | NS |
| DAT1 | SOMS | 154 | 8 | 86 | 60 | 11.9 | 0.0006 |
| DAT1 | MOVS | 139 | 24 | 68 | 47 | 20.86 | <0.0001 |
| DAT2 | control | 294 | 109 | 141 | 44 | ----- | ----- |
| DAT2 | PNDS | 170 | 47 | 91 | 32 | 4.49 | NS |
| DAT2 | RDDS | 161 | 41 | 80 | 40 | 9.82 | 0.0074 |
| DAT2 | OCDS | 131 | 60 | 66 | 5 | 11.6 | 0.003 |
| DAT2 | DEMS | 148 | 36 | 81 | 31 | 7.85 | 0.0197 |
| DAT2 | SOMS | 154 | 32 | 84 | 38 | 14.6 | 0.0007 |
| DAT2 | MOVS | 139 | 41 | 69 | 29 | 3.57 | NS |
| DAT3 | control | 294 | 110 | 138 | 46 | ----- | ----- |
| DAT3 | PNDS | 170 | 75 | 90 | 5 | 17.8 | 0.0001 |
| DAT3 | RDDS | 161 | 64 | 80 | 17 | 2.26 | NS |
| DAT3 | OCDS | 131 | 56 | 56 | 19 | 1.09 | NS |
| DAT3 | DEMS | 148 | 59 | 60 | 29 | 1.96 | NS |
| DAT3 | SOMS | 154 | 80 | 64 | 10 | 8.73 | 0.0127 |
| DAT3 | MOVS | 139 | 62 | 65 | 12 | 48.67 | <0.0001 |
| | | | | | | | |
| SERT1 | control | 294 | 85 | 148 | 61 | ----- | ----- |
| SERT1 | PNDS | 170 | 59 | 92 | 19 | 7.18 | 0.02 |
| SERT1 | RDDS | 161 | 65 | 48 | 48 | 17.8 | 0.0001 |
| SERT1 | OCDS | 131 | 35 | 64 | 32 | 0.76 | NS |
| SERT1 | DEMS | 148 | 28 | 94 | 26 | 7.47 | 0.0239 |
| SERT1 | SOMS | 154 | 58 | 59 | 37 | 6.09 | 0.0476 |
| SERT1 | MOVS | 139 | 21 | 83 | 35 | 9.74 | 0.0077 |
| SERT2 | control | 294 | 120 | 130 | 44 | ----- | ----- |
| SERT2 | PNDS | 170 | 51 | 85 | 34 | 5.82 | 0.05 |
| SERT2 | RDDS | 161 | 41 | 86 | 34 | 11.08 | 0.003 |
| SERT2 | OCDS | 131 | 34 | 70 | 27 | 8.89 | 0.0117 |
| SERT2 | DEMS | 148 | 66 | 71 | 11 | 5.13 | NS |
| SERT2 | SOMS | 154 | 76 | 40 | 38 | 15.75 | 0.0004 |
| SERT2 | MOVS | 139 | 26 | 81 | 32 | 21.0 | <0.0001 |
| SERT3 | control | 294 | 132 | 94 | 68 | ----- | ----- |
| SERT3 | PNDS | 170 | 74 | 57 | 29 | 0.75 | NS |
| SERT3 | RDDS | 161 | 60 | 75 | 26 | 9.87 | 0.0072 |
| SERT3 | OCDS | 131 | 71 | 41 | 16 | 7.13 | 0.0283 |
| SERT3 | DEMS | 148 | 65 | 71 | 12 | 19.04 | <0.0001 |
| SERT3 | SOMS | 154 | 67 | 50 | 37 | 0.09 | NS |
| SERT3 | MOVS | 139 | 62 | 43 | 34 | 0.1 | NS |
| | | | | | | | |
| NET1 | control | 294 | 161 | 112 | 21 | ----- | ----- |
| NET1 | PNDS | 170 | 95 | 69 | 6 | 2.61 | NS |
| NET1 | RDDS | 161 | 90 | 63 | 8 | 0.82 | NS |
| NET1 | OCDS | 131 | 89 | 26 | 16 | 23.4 | <0.0001 |
| NET1 | DEMS | 148 | 82 | 59 | 7 | 0.99 | NS |
| NET1 | SOMS | 154 | 100 | 51 | 3 | 7.57 | 0.0227 |
| NET1 | MOVS | 139 | 55 | 65 | 19 | 10.45 | 0.0054 |
| NET2 | control | 294 | 153 | 119 | 22 | ----- | ----- |
| NET2 | PNDS | 170 | 65 | 85 | 20 | 8.88 | 0.01 |
| NET2 | RDDS | 161 | 39 | 94 | 28 | 35.5 | <0.0001 |
| NET2 | OCDS | 131 | 40 | 55 | 36 | 35.8 | <0.0001 |
| NET2 | DEMS | 148 | 61 | 67 | 20 | 6.69 | 0.0353 |
| NET2 | SOMS | 154 | 86 | 57 | 11 | 0.6 | NS |
| NET2 | MOVS | 139 | 75 | 54 | 10 | 0.14 | NS |
| NET3 | control | 294 | 134 | 127 | 33 | ----- | ----- |
| NET3 | PNDS | 170 | 101 | 86 | 10 | 5.89 | 0.05 |
| NET3 | RDDS | 161 | 45 | 85 | 31 | 15.0 | 0.0005 |
| NET3 | OCDS | 131 | 59 | 63 | 9 | 2.23 | NS |
| NET3 | DEMS | 148 | 83 | 59 | 6 | 8.21 | 0.0165 |
| NET3 | SOMS | 154 | 68 | 76 | 10 | 3.25 | NS |
| NET3 | MOVS | 139 | 59 | 62 | 18 | 0.88 | NS |

| | | | | | | | |
|---|---|---|---|---|---|---|---|
| DAT 1: Genotype 1 = GG, Genotype 2 = GA, Genotype 3 = AA DAT 2: Genotype 1 = AA, Genotype 2 = AT, Genotype 3 = TT DAT 3: Genotype 1 = 10/10, Genotype 2 = 10/non-10, Genotype 3 = non-10/non-10 SERT 1: Genotype 1 = TT, Genotype 2 = TG, Genotype 3 = GG SERT 2: Genotype 1 = LL, Genotype 2 = LS, Genotype 3 = SS SERT 3: Genotype 1= 12/12, Genotype 2= 12/non-12, Genotype 3 = non-12/non-12 NET 1: Genotype 1 = L4/L4, Genotype 2 = L4/S4, Genotype 3 = S4/S4 NET 2: Genotype 1 = AA, Genotype 2 =AT, Genotype 3 = TT NET 3: Genotype 1= TT, Genotype 2 = TC, Genotype 3 = CC NS (not significant) = p Value > 0.05 | | | | | | | |

**Table 2. Inhibition of binding of at human monoamine transporters (Ki in nM) ± mean standard error based on 3 trials**

| Compound | hSERT | hNET | hDAT |
|---|---|---|---|
| mazindol | 56 ± 9 | 24 ± 2 | 37 ± 3.2 |
| Cpd 1 | 101 ± 7.9 | 363 ± 41 | 25 ± 4.8 |
| Cpd 2 | 14 ± 5 | 56 ± 19 | 45 ± 11 |
| Cpd 3 | 312 ± 45 | 89 ± 18 | 111 ± 7.3 |

| | | | |
|---|---|---|---|
| Mazindol: 5-(4-Chlorophenyl)-2,5-dihydro-5H-imidazo(2,1-a)isoindol-5-ol Cpd 1: 5-(3,4-dichlorophenyl)-2,5-dihydro-5H-imidazo(2,1-a)isoindol-5-ol Cpd 2: 5-(4-Chlorophenyl)-2,5-dihydro-5H-imidazo(2,1-a)isoindole Cpd 3: 5-(4-Chlorophenyl)-2,3-dihydro-5H-imidazo(2,1-a)isoindole | | | |

### PCT CLAIMS

According to a first aspect, there is provided a method for treating a neurobehavioral disorder or at least one symptom of such neurobehavioral disorder comprising of administering to a mammal afflicted with a neurobehavioral disorder or symptom thereof a therapeutically effective amount for treating such condition of an isoindole derivative compound of the formula I: 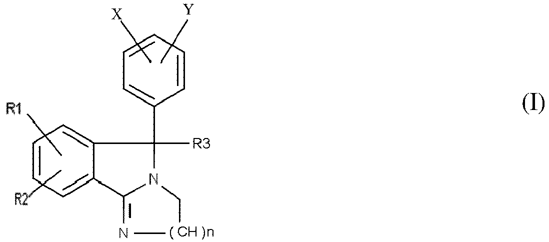 wherein:
n is 1;
R1 and R2 are each independently a member selected from the group consisting of hydrogen, halogen, hydroxy, lower alkyl and lower alkoxy;
R3 is a member selected from the group consisting of hydrogen, halogen, hydroxy, lower alkyl and lower alkoxy;
X and Y are each independently a member selected from the group consiting of hydrogen, halogen, hydroxy, lower alkyl, lower alkoxy and haloalkyl;
or a pharmaceutically acceptable salt, addition compound or pro-drug thereof.

Preferably, the compound of formula I is mazindol. More preferably, the Mazindol is administered in the form of a pro-drug.

In accordance with a second aspect, there is provided a method of treating a neurobehavioral disorder, or at least one symptom of such disorder, in an individual in need thereof which is the result of dysfunction of the tri-monoamine modulating system by the simultaneous action with relatively equal inhibitory effect on all three monoamines comprising administering to the individual an effective amount of a compound according to formula I: 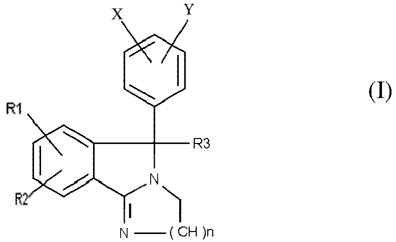 wherein:
n is 1;
R1 and R2 are each independently a member selected from the group consisting of hydrogen, halogen, hydroxy, lower alkyl and lower alkoxy;
R3 is a member selected from the group consisting of hydrogen, halogen, hydroxy, lower alkyl and lower alkoxy;
X and Y are each independently a member selected from the group consiting of hydrogen, halogen, hydroxy, lower alkyl, lower alkoxy and haloalkyl;
or a pharmaceutically acceptable salt, addition compound or pro-drug thereof.

In accordance with a third aspect, there is provided a method for treating a neurobehavioral disorder, or a behavioral symptom of a neurobehavioral disorder, comprising administering to a mammal afflicted with the neurobehavioral disorder a therapeutically effective amount of a compound, or simultaneous combination of compounds, that affects the trimonoamine modulating system by producing a simultaneous and relatively equal inhibitory effect on all three monoamine transporters in the trimonoamine modulating system.

Preferably, the neurobehavioral disorder of the first, second or third aspect is a pervasive developmental disorder, or a behavioral symptom of such disorder.

In a preferred embodiment, the neurobehavioral disorder of the first, second or third aspect is attention deficit hyperactivity disorder.

In an alternative preferred embodiment, the neurobehavioral disorder of the first, second or third aspect is an autism spectrum disorder, or a symptom of such disorder.

In an alternative preferred embodiment, the neurobehavioral disorder of the first, second or third aspect is an anxiety disorder or a behavioral symptom of such disorder.

In an alternative preferred embodiment, the neurobehavioral disorder of the first, second or third aspect is a movement disorder or a symptom of such disorder.

In an alternative preferred embodiment, the neurobehavioral disorder of the first, second or third aspect is an impulse control disorder or a behavioral symptom of such disorder.

In an alternative preferred embodiment, the neurobehavioral disorder of the first, second or third aspect is a personality disorder, or a behavioral symptom of such disorder.

In an alternative preferred embodiment, the neurobehavioral disorder of the first, second or third aspect is a reward deficiency disorder, or a behavioral symptom of such disorder.

In an alternative preferred embodiment, the neurobehavioral disorder of the first, second or third aspect is a somatoform disorder, or a behavioral symptom of such disorder.

In an alternative preferred embodiment, the neurobehavioral disorder of the first, second or third aspect is a dementia disorder, or a behavioral symptom of such disorder.

In an alternative preferred embodiment, the neurobehavioral disorder of the first, second or third aspect is an obsessive compulsive spectrum disorder, or a behavioral symptom of such disorder.

Preferably, the compound of the first, second or third aspect has a Ki at the serotonin, dopamine and norepinephrine transporters in a ratio not greater than 50:1 for any given transporter relative to the other two transporters.

Preferably, said compound of the first, second or third aspect has an IC50 at the serotonin, dopamine and norepinephrine transporters in a ratio not greater than 50:1 for any given transporter relative to the other two transporters.

Preferably, the compound of the first, second or third aspect is administered in conjunction with at least one second drug compound selected from the group consisting of adrenergics, adrenocortical steroids, adrenocortical suppressants, aldosterone antagonists, amino acids, analeptics, analgesics, anorectic compounds, anorexics, anti-anxiety agents, antidepressants, antihypertensives, anti-inflammatorys, antinauseants, antineutropenics, antiobsessional agents, antiparkinsonians, antipsychotics, appetite suppressants, blood glucose regulators, carbonic anhydrase inhibitors, cardiotonics, cardiovascular agents, choleretics, cholinergics, cholinergic agonists, cholinesterase deactivators, cognition adjuvants, cognition enhancers, hormones, memory adjuvants, mental performance enhancers, mood regulators, neuroleptics, neuroprotectives, psychotropics, relaxants, sedative-hypnotics, serotonin antagonists, serotonin inhibitors, serotonin receptor antagonists, stimulants, thyroid hormones, thyroid inhibitors, thyromimetics, cerebral ischemia agents, vasoconstrictors and vasodilators.

Preferably, the compound of formula I of the first, second or third aspect is administered in the form of a pro-drug.

In accordance with a fourth aspect, there is provided a method for the prevention and/or curative treatment of attention deficit hyperactivity disorder, (ADHD) or at least one of the symptoms associated with ADHD comprising administering to a patient in need thereof an effective amount of mazindol.

Preferably, the patient is an infant, child, adolescent or adult.

Preferably,the administration is oral, anal, parenteral, intramuscular or intravenous.

Preferably,the at least one symptom is selected from the group consisting of inattention, impulsivity, impatience, diurnal or noctournal hyperactivity, insomnia and restless legs syndrome.

Preferably, in the method of the fourth aspect mazindol is administered in a daily dose between 1 and 2 mg.

Preferably, in the method of any one of the first, second, third or fourth aspects mazindol is administered in a daily dose between 1 and 2 mg.

In accordance with a fifth aspect, there is provided use of an isoindole derivative compound of formula I: 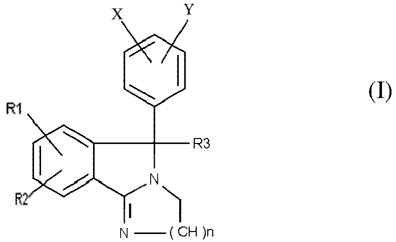 wherein:
n is 1;
R1 and R2 are each independently a member selected from the group consisting of hydrogen, halogen, hydroxy, lower alkyl and lower alkoxy;
R3 is a member selected from the group consisting of hydrogen, halogen, hydroxy, lower alkyl and lower alkoxy;
X and Y are each independently a member selected from the group consiting of hydrogen, halogen, hydroxy, lower alkyl, lower alkoxy and haloalkyl;
or a pharmaceutically acceptable salt, addition compound or pro-drug therof,
for the preparation of a medicament to treat an individual with a neurobehavioral disorder.

Preferably, the compound of formula I is mazindol.

In accordance with a sixth aspect, there is provided use of a compound according to formula I: 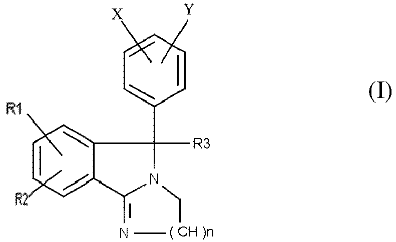 wherein:
n is 1;
R1 and R2 are each independently a member selected from the group consisting of hydrogen, halogen, hydroxy, lower alkyl and lower alkoxy;
R3 is a member selected from the group consisting of hydrogen, halogen, hydroxy, lower alkyl and lower alkoxy;
X and Y are each independently a member selected from the group consiting of hydrogen, halogen, hydroxy, lower alkyl, lower alkoxy and haloalkyl;
or a pharmaceutically acceptable salt, addition compound or pro-drug therof,
for preparation of a medicament that simultaneously acts on all three monoamines with relatively equal inhibitory effect to treat an individual in need thereof having a neurobehavioral disorder, or at least one symptom of such disorder, which results from dysfunction of the tri-monoamine modulating system.

In accordance with a seventh aspect, there is provided use of a compound for the preparation of a medicament that produces a simultaneous and relatively equal inhibitory effect on all three monoamine transporters in the trimonoamine modulating system for the treatment of an individual with a neurobehavioral disorder, or at least one symptom of such disorder.

Preferably, the neurobehavioral disorder of the fifth, sixth or seventh aspect is a pervasive developmental disorder, or a behavioral symptom of such disorder. More preferably, said wherein the neurobehavioral disorder is attention deficit hyperactivity disorder.

Preferably, the neurobehavioral disorder of the fifth, sixth or seventh aspect is an autism spectrum disorder, an anxiety disorder, a movement disorder, an impulse control disorder, a personality disorder, a reward deficiency disorder, a somatoform disorder, a dementia disorder and an obsessive compulsive spectrum disorder, or a symptom of such a disorder.

Preferably, the compound of the fifth, sixth or seventh aspect has a Ki at the serotonin, dopamine and norepinephrine transporters in a ratio not greater than 50:1 for any given transporter relative to the other two transporters.

Preferably, said compound of the fifth, sixth or seventh aspect has an IC50 at the serotonin, dopamine and norepinephrine transporters in a ratio not greater than 50:1 for any given transporter relative to the other two transporters.

Preferably, the compound of the fifth, sixth or seventh aspect is administered in conjunction with at least one second drug compound selected from the group consisting of adrenergics, adrenocortical steroids, adrenocortical suppressants, aldosterone antagonists, amino acids, analeptics, analgesics, anorectic compounds, anorexics, anti-anxiety agents, antidepressants, antihypertensives, anti-inflammatorys, antinauseants, antineutropenics, antiobsessional agents, antiparkinsonians, antipsychotics, appetite suppressants, blood glucose regulators, carbonic anhydrase inhibitors, cardiotonics, cardiovascular agents, choleretics, cholinergics, cholinergic agonists, cholinesterase deactivators, cognition adjuvants, cognition enhancers, hormones, memory adjuvants, mental performance enhancers, mood regulators, neuroleptics, neuroprotectives, psychotropics, relaxants, sedative-hypnotics, serotonin antagonists, serotonin inhibitors, serotonin receptor antagonists, stimulants, thyroid hormones, thyroid inhibitors, thyromimetics, cerebral ischemia agents, vasoconstrictors and vasodilators.

Preferably, the compound of formula I of the fifth, sixth or seventh aspect is administered in the form of a pro-drug.

In accordance with an eighth aspect, there is provided use of mazindol for preparation of a medicament for preventive and/or curative treatment of a patient having attention deficit hyperactivity disorder or the symptoms associated therewith.

Preferably, the patient is an infant, child, adolescent or adult.

Preferably the administration is oral, anal, parenteral, intramuscular or intravenous.

Preferably, the at least one symptom is selected from the group consisting of inattention, impulsivity, impatience, diurnal or noctournal hyperactivity, insomnia and restless legs syndrome.

Preferably, mazindol is administered in a daily dose between 1 and 2 mg.

In accordance with a ninth aspect, there is provided an isoindole derivative compound of formula I: 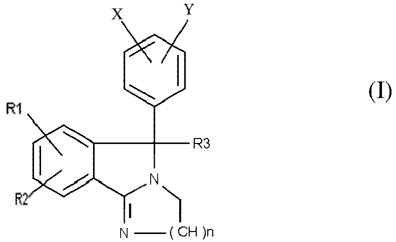 wherein:
n is 1;
R1 and R2 are each independently a member selected from the group consisting of hydrogen, halogen, hydroxy, lower alkyl and lower alkoxy;
R3 is a member selected from the group consisting of hydrogen, halogen, hydroxy, lower alkyl and lower alkoxy;
X and Y are each independently a member selected from the group consiting of hydrogen, halogen, hydroxy, lower alkyl, lower alkoxy and haloalkyl;
or a pharmaceutically acceptable salt, addition compound or pro-drug therof,
for the treatment of a neurobehavioral disorder or at least one symptom of such disorder.

Preferably, the compound of formula I is mazindol.

In accordance with a tenth aspect, there is provided a compound according to formula I: 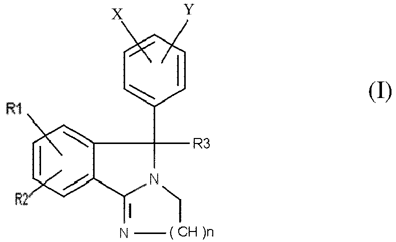 wherein:
n is 1;
R1 and R2 are each independently a member selected from the group consisting of hydrogen, halogen, hydroxy, lower alkyl and lower alkoxy;
R3 is a member selected from the group consisting of hydrogen, halogen, hydroxy, lower alkyl and lower alkoxy;
X and Y are each independently a member selected from the group consiting of hydrogen, halogen, hydroxy, lower alkyl, lower alkoxy and haloalkyl;
or a pharmaceutically acceptable salt, addition compound or pro-drug therof,
that simultaneously acts on all three monoamines with relatively equal inhibitory effect for the treatment of a neurobehavioral disorder, or at least one symptom of such disorder, which results from dysfunction of the tri-monoamine modulating system.

In accordance with an eleventh aspect, there is provided a compound that produces a simultaneous and relatively equal inhibitory effect on all three monoamine transporters in the trimonoamine modulating system for the treatment ofh a neurobehavioral disorder, or at least one symptom of such disorder.

Preferably, the neurobehavioral disorder of the ninth, tenth or eleventh aspect is a pervasive developmental disorder, or a behavioral symptom of such disorder. More preferably, the neurobehavioral disorder is attention deficit hyperactivity disorder.

Preferably, the neurobehavioral disorder of the ninth, tenth or eleventh aspect is an autism spectrum disorder, an anxiety disorder, a movement disorder, an impulse control disorder, a personality disorder, a reward deficiency disorder, a somatoform disorder, a dementia disorder and an obsessive compulsive spectrum disorder, or a symptom of such a disorder.

Preferably, the compound of the ninth, tenth or eleventh aspect has a Ki at the serotonin, dopamine and norepinephrine transporters in a ratio not greater than 50:1 for any given transporter relative to the other two transporters.

Preferably, said compound of the ninth, tenth or eleventh aspect has an IC50 at the serotonin, dopamine and norepinephrine transporters in a ratio not greater than 50:1 for any given transporter relative to the other two transporters.

Preferably, the compound of the ninth, tenth or eleventh aspect is administered in conjunction with at least one second drug compound selected from the group consisting of adrenergics, adrenocortical steroids, adrenocortical suppressants, aldosterone antagonists, amino acids, analeptics, analgesics, anorectic compounds, anorexics, anti-anxiety agents, antidepressants, antihypertensives, anti-inflammatorys, antinauseants, antineutropenics, antiobsessional agents, antiparkinsonians, antipsychotics, appetite suppressants, blood glucose regulators, carbonic anhydrase inhibitors, cardiotonics, cardiovascular agents, choleretics, cholinergics, cholinergic agonists, cholinesterase deactivators, cognition adjuvants, cognition enhancers, hormones, memory adjuvants, mental performance enhancers, mood regulators, neuroleptics, neuroprotectives, psychotropics, relaxants, sedative-hypnotics, serotonin antagonists, serotonin inhibitors, serotonin receptor antagonists, stimulants, thyroid hormones, thyroid inhibitors, thyromimetics, cerebral ischemia agents, vasoconstrictors and vasodilators.

Preferably, the compound of formula I of the ninth, tenth or eleventh aspect is administered in the form of a pro-drug.

In accordance with an twelfth aspect, there is provided the compound of mazindol for the preventive and/or curative treatment of a patient having attention deficit hyperactivity disorder or the symptoms associated therewith.

Preferably, the patient is an infant, child, adolescent or adult.

Preferably, the administration is oral, anal, parenteral, intramuscular or intravenous.

Preferably, the at least one symptom is selected from the group consisting of inattention, impulsivity, impatience, diurnal or noctournal hyperactivity, insomnia and restless legs syndrome.

Preferably, mazindol is administered in a daily dose between 1 and 2 mg.

In accordance with an thirteenth aspect, there is provided a pharmaceutical preparation comprising mazindol, optionally in combination with at least one additional active ingredient and/or pharmaceutically acceptable excipient, for use in treating attention deficit hyperactivity disorder or its symptoms.

In accordance with an fourteenth aspect, there is provided a pharmaceutical preparation comprising mazindol, optionally in combination with at least one additional active ingredient and/or pharmaceutically acceptable excipients, for use in treating neurobehavioral disorders resulting from dysfunction of the trimonoamine modulating system.

## Claims

1. Mazindol, or a pharmaceutically acceptable salt thereof, for use in treating inattention and/or hyperactivity caused by attention deficient hyperactivity disorder (ADHD) in a mammalian patient.

2. Mazindol, or a pharmaceutically acceptable salt thereof, for use according to claim 1, wherein the mazindol is for use in treating inattention and hyperactivity caused by attention deficient hyperactivity disorder (ADHD) in a mammalian patient.

3. Mazindol, or a pharmaceutically acceptable salt thereof, for use according to either claim 1 or claim 2, wherein the mazindol is for use in treating impulsivity caused by attention deficient hyperactivity disorder (ADHD) in a mammalian patient.

4. Mazindol, or a pharmaceutically acceptable salt thereof, for use according to any preceding claim, wherein the daily dose of mazindol is 0.001 mg/kg to 200 mg/kg.

5. Mazindol, or a pharmaceutically acceptable salt thereof, for use according to claim 4, wherein the mazindol is administered in a daily dose between 1 and 2 mg.

6. Mazindol, or a pharmaceutically acceptable salt thereof, for use according to any preceding claim, wherein the patient is an infant, child, adolescent or adult.

7. Mazindol, or a pharmaceutically acceptable salt thereof, for use according to any preceding claim, wherein the administration is oral, anal, parenteral, intramuscular or intravenous.
